# EUROPEAN PATENT SPECIFICATION

(11) **EP 0 591 462 B1**
(45) Date of publication and mention of the grant of the patent: **16.04.2003**
(21) Application number: 92915715.4
(22) Date of filing: 14.07.1992
(51) Int. Cl.: C12N 15/00, C12N 15/12, A01K 67/027, C12N 5/16, C12N 5/22, C12N 15/87, A61L 27/00, C07K 2/00

(54) **UNIVERSAL DONOR CELLS**
UNIVERSALE SPENDERZELLEN
CELLULES DONNEUSES UNIVERSELLES

(30) Priority: 15.07.1991 US 729926; 29.06.1992 US 906394
(43) Date of publication of application: 13.04.1994
(60) Divisional application: 00114262.9
(73) Proprietor: Oklahoma Medical Research Foundation, Oklahoma City Oklahoma 73104 (US); Yale University, New Haven, CT 06500 (US)
(72) Inventor: SIMMS, Peter, J., Del Mar, CA 92014-3614 (US); BOTHWELL, Alfred, L., M., Guilford, CT 06437 (US); ELLIOT, Eileen, A., New Haven, CT 06511 (US); FLAVELL, Richard, A., Guildford, CT 06437 (US); MADRI, Joseph, North Branford, CT 06417 (US); ROLLINS, Scott, Cheshire, CT 06410 (US); BELL, Leonard, Woodbridge, CT 06525 (US); SQUINTO, Stephen, Irvington, NY 10533 (US)
(74) Representative: Baldock, Sharon Claire
(86) International application number: US9205920
(87) International publication number: WO93002188

(56) References cited:
- EP-A- 0 244 267
- EP-A- 0 406 665
- WO-A-89/03875
- WO-A-91/01140
- WO-A-91/02002
- WO-A-91/05855
- WO-A-91/18097
- WO-A-92/09688
- NATURE vol. 342, no. 6248, 23 November 1989, UK pages 435 - 438 ZIJLSTRA, M. ET AL. 'GERM-LINE TRANSMISSION OF A DISRUPTED BETA2-MICROGLOBULIN GENE PRODUCED BY HOMOLOGOUS RECOMBINATION IN EMBRYONIC STEM CELLS' cited in the application
- JOURNAL OF BIOCHEMISTRY. vol. 266, no. 20, 15 July 1991, TOKYO JP pages 13418 - 13422 ZHAO, J. ET AL. 'Amplified gene expression in CD59-transfected Chinese Hamster Ovary Cells confers protection against the membrane attack complex of human complement'
- MEDICAL PROGRESS THROUGH TECHNOLOGY vol. 14, no. 3/4, 1989, DORDRECHT, NL pages 115 - 163 HILBERT, S. ET AL. 'Tissue-derived biomaterials and their use in cardiovascular prosthetic devices'
- 'The Trials of Xenotransplantation' NATURE vol. 406, no. 6797, 17 August 2000, page 661
- ARTIP J H ET AL: 'Cardiac Xenotransplantation' CURRENT OPINION IN CARDIOLOGY vol. 12, 1997, pages 172 - 178
- VANHOVE B ET AL: 'Intracellular Expression in Pig Cells...' TRANSPLANTATION vol. 66, no. 11, 15 December 1998, pages 1477 - 148-
- SANDRIN M S ETAL: 'Deduction of the Major Porcine Xenoantigen ...' XENOTRANSPLANTATION vol. 3, 1996, pages 134 - 140-
- LAI L ET AL:" Production of alpha-1,3-Galactosyl transferase Knockout Pigs by Nuclear Transfer Cloning" , Sciencexpress, published online 03.01.2002
- Süddeutsche Zeitung, 08.01.2002, No. 6, page V2/7: "Neun Schweinchen namens Hope"

### SEQUENCE LISTING

(1) GENERAL INFORMATION:
   (i) APPLICANT: Sims, Peter J.
      Bothwell, Alfred L.M.
      Elliott, Eileen A.
      Flavell, Richard A.
      Madri, Joseph
      Rollins, Scott
      Bell, Leonard
      Squinto, Stephen
   (ii) TITLE OF INVENTION: Universal Donor Cells
   (iii) NUMBER OF SEQUENCES: 4
   (iv) CORRESPONDENCE ADDRESS:
      (A) ADDRESSEE: Kilpatrick & Cody
      (B) STREET: 1100 Peachtree Street, Suite 2800
      (C) CITY: Atlanta
      (D) STATE:, Georgia
      (E) COUNTRY: U.S.
      (F) ZIP: 30309-4530
   (V) COMPUTER READABLE FORM:
      (A) MEDIUM TYPE: Floppy disk
      (B) COMPUTER: IBM PC compatible
      (C) OPERATING SYSTEM: PC-DOS/MS-DOS
      (D) SOFTWARE: PatentIn Release #1.0, Version #1.25
   (vi) CURRENT APPLICATION DATA:
      (A) APPLICATION NUMBER: US
      (B) FILING DATE:
      (C) CLASSIFICATION:
   (viii) ATTORNEY/AGENT INFORMATION:
      (A) NAME: Pabst, Patrea L.
      (B) REGISTRATION NUMBER: 31,284
      (C) REFERENCE/DOCKET NUMBER: OMRF135
   (ix) TELECOMMUNICATION INFORMATION:
      (A) TELEPHONE: 404-815-6500
      (B) TELEFAX: 404-815-6555
(2) INFORMATION FOR SEQ ID NO:1:
   (i) SEQUENCE CHARACTERISTICS:
      (A) LENGTH: 2124 base pairs
      (B) TYPE: nucleic acid
      (C) STRANDEDNESS: single
      (D) TOPOLOGY: linear
   (ii) MOLECULE TYPE: cDNA
   (iii) HYPOTHETICAL: NO
   (iv) ANTI-SENSE: NO
   (vi) ORIGINAL SOURCE:
      (A) ORGANISM: Homo sapiens
   (vii) IMMEDIATE SOURCE:
      (A) LIBRARY: GenBank HUMCD46Q
      (B) CLONE: HUMCD46 cDNA
   (xi) SEQUENCE DESCRIPTION: SEQ ID NO:1:
   GAATTCGGGG ATAACAGCGT CTTCCGCGCC GCGCATGGAG CCTCCCGGCC GCCGCGAGTG 60
   TCCCTTTCCT TCCTGGCGCT TTCCTGGGTT GCTTCTGGCG GCCATGGTGT TGCTGCTGTA 120
   CTCCTTCTCC GATGCCTGTG AGGAGCCACC AACATTTGAA GCTATGGAGC TCATTGGTAA 180
   ACCAAAACCC TACTATGAGA TTGGTGAACG AGTAGATTAT AAGTGTAAAA AAGGATACTT 240
   CTATATACCT CCTCTTGCCA CCCATACTAT TTGTGATCGG AATCATACAT GGCTACCTGT 300
   CTCAGATGAC GCCTGTTATA GAGAAACATG TCCATATATA CGGGATCCTT TAAATGGCCA 360
   AGCAGTCCCT GCAAATGGGA CTTACGAGTT TGGTTATCAG ATGCACTTTA TTTGTAATGA 420
   GGGTTATTAC TTAATTGGTG AAGAAATTCT ATATTGTGAA CTTAAAGGAT CAGTAGCAAT 480
   TTGGAGCGGT AAGCCCCCAA TATGTGAAAA GGTTTTGTGT ACACCACCTC CAAAAATAAA 540
   AAATGGAAAA CACACCTTTA GTGAAGTAGA AGTATTTGAG TATCTTGATG CAGTAACTTA 600
   TAGTTGTGAT CCTGCACCTG GACCAGATCC ATTTTCACTT ATTGGAGAGA GCACGATTTA 660
   TTGTGGTGAC AATTCAGTGT GGAGTCGTGC TGCTCCAGAG TGTAAAGTGG TCAAATGTCG 720
   ATTTCCAGTA GTCGAAAATG GAAAACAGAT ATCAGGATTT GGAAAAAAAT TTTACTACAA 780
   AGCAACAGTT ATGTTTGAAT GCGATAAGGG TTTTTACCTC GATGGCAGCG ACACAATTGT 840
   CTGTGACAGT AACAGTACTT GGGATCCCCC AGTTCCAAAG TGTCTTAAAG TGTCGACTTC 900
   TTCCACTACA AAATCTCCAG CGTCCAGTGC CTCAGGTCCT AGGCCTACTT ACAAGCCTCC 960
   AGTCTCAAAT TATCCAGGAT ATCCTAAACC TGAGGAAGGA ATACTTGACA GTTTGGATGT 1020
   TTGGGTCATT GCTGTGATTG TTATTGCCAT AGTTGTTGGA GTTGCAGTAA TTTGTGTTGT 1080
   CCCGTACAGA TATCTTCAAA GGAGGAAGAA GAAAGGCACA TACCTAACTG ATGAGACCCA 1140
   CAGAGAAGTA AAATTTACTT CTCTCTGAGA AGGAGAGATG AGAGAAAGGT TTGCTTTTAT 1200
   CATTAAAAGG AAAGCAGATG GTGGAGCTGA ATATGCCACT TACCAGACTA AATCAACCAC 1260
   TCCAGCAGAG CAGAGAGGCT GAATAGATTC CACAACCTGG TTTGCCAGTT CATCTTTTGA 1320
   CTCTATTAAA ATCTTCAATA GTTGTTATTC TGTAGTTTCA CTCTCATGAG TGCAACTGTG 1380
   GCTTAGCTAA TATTGCAATG TGGCTTGAAT GTAGGTAGCA TCCTTTGATG CTTCTTTGAA 1440
   ACTTGTATGA ATTTGGGTAT GAACAGATTG CCTGCTTTCC CTTAAATAAC ACTTAGATTT 1500
   ATTGGACCAG TCAGCACAGC ATGCCTGGTT GTATTAAAGC AGGGATATGC TGTATTTTAT 1560
   AAAATTGGCA AAATTAGAGA AATATAGTTC ACAATGAAAT TATATTTTCT TTGTAAAGAA 1620
   AGTGGCTTGA AATCTTTTTT GTTCAAAGAT TAATGCCAAC TCTTAAGATT ATTCTTTCAC 1680
   CAACTATAGA ATGTATTTTA TATATCGTTC ATTGTAAAAA GCCCTTAAAA ATATGTGTAT 1740
   ACTACTTTGG CTCTTGTGCA TAAAAACAAG AACACTGAAA ATTGGGAATA TGCACAAACT 1800
   TGGCTTCTTT AACCAAGAAT ATTATTGGAA AATTCTCTAA AAGTAAAGGG TAAATTCTCT 1860
   ATTTTTTGTA ATGTGTTCGG TGATTTCAGA AAGCTAGAAA GTGTATGTGT GGCATTTGTT 1920
   TTCACTTTTT AAAACATCCC TAACTGATCG AATATATCAG TAATTTCAGA ATCAGATGCA 1980
   TCCTTTCATA AGAAGTGAGA GGACTCTGAC AGCCATAACA GGAGTGCCAC TTCATGGTGC 2040
   GAAGTGAACA CTGTAGTCTT GTTGTTTTCC CAAAGAGAAC TCCGTATGTT CTCTTAGGTT 2100
   GAGTAACCCA CTCTGCCCGA ATTC 2124
(2) INFORMATION FOR SEQ ID NO:2:
   (i) SEQUENCE CHARACTERISTICS:
      (A) LENGTH: 2847 base pairs
      (B) TYPE: nucleic acid
      (C) STRANDEDNESS: single
      (D) TOPOLOGY: linear
   (ii) MOLECULE TYPE: cDNA
   (iii) HYPOTHETICAL: NO
   (iv) ANTI-SENSE: NO
   (vi) ORIGINAL SOURCE:
      (A) ORGANISM: Homo sapiens
   (vii) IMMEDIATE SOURCE:
      (A) LIBRARY: GenBank HUMDAF; HUMDAFC1
      (B) CLONE: Human DAF cDNA
   (ix) FEATURE:
      (A) NAME/KEY: misc feature
      (B) LOCATION: 1..819
      (D) OTHER INFORMATION: /note= "HUMDAFC1 (Promotor and 5' end of Exon 1, genomic sequence)"
   (xi) SEQUENCE DESCRIPTION: SEQ ID NO:2:
   TTCTCTCTAC AGTCAGTCTG GAGTAATCCC AAAGTGGTGT CTTTCGTAAA TAAGGAGAAC 60
   CCGGGTGAAG AAAATGACTC CCACCCGAAC AAGGCATGAA CAATGTTCAC TCCCTACTGT 120
   GTTATTCAAC CTGTTTCCCC AGGTCTCTGT TTTCACATTA GAGAGTGTTC TAGGAGATGA 180
   CGCCCTTCCT CCTTAGTTAT TTCCCCACCC TCGTGCTGGC CTTTGACAGA CCTCCCAGTA 240
   GAGGGCCCAA GACGCGGGTA GAGCACCGCG TCTCAGCGCC TGAGTCTCAG CCCCCGAACT 300
   CCACCGCACC TCGAGGTCCC CTTGGCACGA CTCAAGCGCG GGGATGCTCC GCTTAGACGA 360
   ACTCACGTGC GGGCAGCAAG GCCTGCGATA CTTGAGCACC CCTCCCCCTC TCCCGTTTAC 420
   ACCCCGTTTG TGTTTACGTA GCGAGGAGAT ATTTAGGTTT CTAGAAGGCA GGTCATCGCA 480
   GGCCCCACCC AGCAGTGGAG AGAGTGAGTC CAGAGGGTGT TGCCAGGAGC TCCTCCTCCT 540
   TCCCCTCCCC ACTCTCCCCG AGTCTAGGGC CCCGGGGTAT GACGCCGGAG CCCTCTGACC 600
   GCACCTCTGA CCACAACAAA CCCCTACTCC ACCCGTCTTG TTTGTCCCAC CCTTGGTGAC 660
   GCAGAGCCCC AGCCCAGACC CCGCCCAAAG CACTCATTTA ACTGGTATTG CGGAGCCACG 720
   AGGCTTCTGA CTTACTGCAA CTCGCTCCGG CCGCTGGGCG TAGCTGCGAC TCGGCGGAGT 780
   CCCGGCGGCG CGTCCTTGTT CTAACCCGGC GCGCCATGAC CGTCGCGCGG CCGAGCGTGC 840
   CCGCGGCGCT GCCCCTCCTC GGGGAGCTGC CCCGGCTGCT GCTGCTGGTG CTGTTGTGCC 900
   TGCCGGCCGT GTGGGGTGAC TGTGGCCTTC CCCCAGATGT ACCTAATGCC CAGCCAGCTT 960
   TGGAAGGCCG TACAAGTTTT CCCGAGGATA CTGTAATAAC GTACAAATGT GAAGAAAGCT 1020
   TTGTGAAAAT TCCTGGCGAG AAGGACTCAG TGACCTGCCT TAAGGGCATG CAATGGTCAG 1080
   ATATTGAAGA GTTCTGCAAT CGTAGCTGCG AGGTGCCAAC AAGGCTAAAT TCTGCATCCC 1140
   TCAAACAGCC TTATATCACT CAGAATTATT TTCCAGTCGG TACTGTTGTG GAATATGAGT 1200
   GCCGTCCAGG TTACAGAAGA GAACCTTCTC TATCACCAAA ACTAACTTGC CTTCAGAATT 1260
   TAAAATGGTC CACAGCAGTC GAATTTTGTA AAAAGAAATC ATGCCCTAAT CCGGGAGAAA 1320
   TACGAAATGG TCAGATTGAT GTACCAGGTG GCATATTATT TGGTGCAACC ATCTCCTTCT 1380
   CATGTAACAC AGGGTACAAA TTATTTGGCT CGACTTCTAG TTTTTGTCTT ATTTCAGGCA 1440
   GCTCTGTCCA GTGGAGTGAC CCGTTGCCAG AGTGCAGAGA AATTTATTGT CCAGCACCAC 1500
   CACAAATTGA CAATGGAATA ATTCAAGGGG AACGTGACCA TTATGGATAT AGACAGTCTG 1560
   TAACGTATGC ATGTAATAAA GGATTCACCA TGATTGGAGA GCACTCTATT TATTGTACTG 1620
   TGAATAATGA TGAAGGAGAG TGGAGTGGCC CACCACCTGA ATGCAGAGGA AAATCTCTAA 1680
   CTTCCAAGGT CCCACCAACA GTTCAGAAAC CTACCACAGT AAATGTTCCA ACTACAGAAG 1740
   TCTCACCAAC TTCTCAGAAA ACCACCACAA AAACCACCAC ACCAAATGCT CAAGCAACAC 1800
   GGAGTACACC TGTTTCCAGG ACAACCAAGC ATTTTCATGA AACAACCCCA AATAAAGGAA 1860
   GTGGAACCAC TTCAGGTACT ACCCGTCTTC TATCTGGGCA CACGTGTTTC ACGTTGACAG 1920
   GTTTGCTTGG GACGCTAGTA ACCATGGGCT TGCTGACTTA GCCAAAGAAG AGTTAAGAAG 1980
   AAAATACACA CAAGTATACA GACTGTTCCT AGTTTCTTAG ACTTATCTGC ATATTGGATA 2040
   AAATAAATGC AATTGTGCTC TTCATTTAGG ATGCTTTCAT TGTCTTTAAG ATGTGTTAGG 2100
   AATGTCAACA GAGCAAGGAG AAAAAAGGCA GTCCTGGAAT CACATTCTTA GCACACCTGC 2160
   GCCTCTTGAA AATAGAACAA CTTGCAGAAT TGAGAGTGAT TCCTTTCCTA AAAGTGTAAG 2220
   AAAGCATAGA GATTTGTTCG TATTAAGAAT GGGATCACGA GGAAAAGAGA AGGAAAGTGA 2280
   TTTTTTTCCA CAAGATCTGA AATGATATTT CCACTTATAA AGGAAATAAA AAATGAAAAA 2340
   CATTATTTGG ATATCAAAAG CAAATAAAAA CCCAATTCAG TCTCTTCTAA GCAAAATTGC 2400
   TAAAGAGAGA TGACCACATT ATAAAGTAAT CTTTGGCTAA GGCATTTTCA TCTTTCCTTC 2460
   GGTTGGCAAA ATATTTTAAA GGTAAAACAT GCTGGTGAAC CAGGGTGTTG ATGGTGATAA 2520
   GGGAGGAATA TAGAATGAAA GACTGAATCT TCCTTTGTTG CACAAATAGA GTTTGGAAAA 2580
   AGCCTGTGAA AGGTGTCTTC TTTGACTTAA TGTCTTTAAA AGTATCCAGA GATACTACAA 2640
   TATTAACATA AGAAAAGATT ATATATTATT TCTGAATCGA GATGTCCATA GTCAAATTTG 2700
   TAAATCTTAT TCTTTTGTAA TATTTATTTA TATTTATTTA TGACAGTGAA CATTCTGATT 2760
   TTACATGTAA AACAAGAAAA GTTGAAGAAG ATATGTGAAG AAAAATGTAT TTTTCCTAAA 2820
   TAGAAATAAA TGATCCCATT TTTTGGT 2847
(2) INFORMATION FOR SEQ ID NO:3:
   (i) SEQUENCE CHARACTERISTICS:
      (A) LENGTH: 103 amino acids
      (B) TYPE: amino acid
      (C) STRANDEDNESS: single
      (D) TOPOLOGY: linear
   (ii) MOLECULE TYPE: protein
   (iii) HYPOTHETICAL: NO
   (iv) ANTI-SENSE: NO
   (V) FRAGMENT TYPE: N-terminal
   (vi) ORIGINAL SOURCE:
      (A) ORGANISM: Homo sapiens
   (vii) IMMEDIATE SOURCE:
      (B) CLONE: CD59
   (Xi) SEQUENCE DESCRIPTION: SEQ ID NO:3:
   Leu Gln Cys Tyr Asn Cys Pro Asn Pro Thr Ala Asp Cys Lys Thr Ala
   1               5                   10                  15
   Val Asn Cys Ser Ser Asp Phe Asp Ala Cys Leu Ile Thr Lys Ala Gly
               20                  25                  30
   Leu Gln Val Tyr Asn Lys Cys Trp Lys Phe Glu His Cys Asn Phe Asn
           35                  40                  45
   Asp Val Thr Thr Arg Leu Arg Glu Asn Glu Leu Thr Tyr Tyr Cys Cys
       50                  55                  60
   Lys Lys Asp Leu Cys Asn Phe Asn Glu Gln Leu Glu Asn Gly Gly Thr
   65                  70                  75                  80
   Ser Leu Ser Glu Lys Thr Val Leu Leu Leu Val Thr Pro Phe Leu Ala
                   85                  90                  95
   Ala Ala Trp Ser Leu His Pro
               100
(2) INFORMATION FOR SEQ ID NO:4:
   (i) SEQUENCE CHARACTERISTICS:
      (A) LENGTH: 315 base pairs
      (B) TYPE: nucleic acid
      (C) STRANDEDNESS: single
      (D) TOPOLOGY: linear
   (ii) MOLECULE TYPE: CDNA
   (iii) HYPOTHETICAL: NO
   (iv) ANTI-SENSE: NO
   (vi) ORIGINAL SOURCE:
      (A) ORGANISM: Homo sapiens
   (vii) IMMEDIATE SOURCE:
      (B) CLONE: CD59
   (xi) SEQUENCE DESCRIPTION: SEQ ID NO:4:
   CTGCAGTGCT ACAACTGTCC TAACCCAACT GCTGACTGCA AAACAGCCGT CAATTGTTCA     60
   TCTGATTTTG ATGCGTGTCT CATTACCAAA GCTGGGTTAC AAGTGTATAA CAAGTGTTGG    120
   AAGTTTGAGC ATTGCAATTT CAACGACGTC ACAACCCGCT TGAGGGAAAA TGAGCTAACG    180
   TACTACTGCT GCAAGAAGGA CCTGTGTAAC TTTAACGAAC AGCTTGAAAA TGGTGGGACA    240
   TCCTTATCAG AGAAAACAGT TCTTCTGCTG GTGACTCCAT TTCTGGCAGC AGCCTGGAGC    300
   CTTCATCCCT AAGTC                                                     315

## Description

### Background of the Invention

This invention relates to genetically engineered endothelial cells and, in particular, to endothelial cells which have been modified to resist lysis by complement and evade the host's immune mechanisms for removing foreign cells, when inserted into a non-autologous host.

Endothelial cells are specialized cells which form the lining of the heart and the blood vessels. Because of their direct contact with the circulating blood, a number of proposals have been made to genetically engineer these cells and use them as *"in vivo*" drug delivery systems, for example, by Culliton, B, J. 1989. "Designing Cells to Deliver Drags," Science 246:746-751; and Zwiebel et al., "High-Level Recombinant Gene Expression in Rabbit Endothelial Cells Transduced by Retroviral Vectors," Science 243:220-222 (transfer of a human adenosine deaminase gene and a rat growth hormone gene to aortic endothelial cells using a retroviral vector and demonstration of the secretion of rat growth hormone from such cells after seeding onto a synthetic vascular graft).

Natural endothelial cells play important roles in normal physiology. In particular, these cells constitute the interface between the blood and the vessel wall and the organs of the body. As such, endothelial cells secrete various natural products directly into the blood stream, maintain an antithrombotic surface on the inside of the vessel, restrict leukocytes from penetrating the vessel wall, regulate various of the biological properties of smooth muscle cells, and participate in the control of vessel wall tone. Therefore, loss of endothelial cells results in the loss of these normal physiological processes and ultimately leads to pathological conditions such as coronary artery disease, organ transplant rejection and vasculitis.

Accordingly, in addition to their use as a medium for the *in vivo* administration of therapeutics, there is a need to provide genetically engineered endothelial cells to replace natural endothelial cells which have been lost due to disease or surgery.

In the past, proposals and/or efforts to use endothelial cells for either administration of therapeutics or cell replacement have generally been limited to autologous cells, i.e., cells derived from the organism undergoing treatment. Alternatively, the patient must be immunosuppressed, which is costly and leaves the patient vulnerable to infection.

This approach has suffered from a number of problems. For example, it is difficult to harvest healthy endothelial cells from the individual to be treated in significant quantities. The procedures for doing so require removal of a section of vasculature and then scraping or otherwise dislodging the endothelial cells from the walls of the vessels. As a result, to be useful for either the administration of therapeutics or cell replacement, a large number of autologous endothelial cells must be grown in culture. To be of practical use, especially in the case of cell replacement, this culturing must take place quickly. Unfortunately, the cell doubling time for endothelial cells is on the order of at least 24 to 48 hours, leading to time periods on the order of a week or more before sufficient quantities of endothelial cells are available for genetic engineering or cell replacement. In addition, under normal physiological conditions, the cell doubling time for natural endothelial cells *in vivo* is also prolonged, making naturally occurring cell replacement *in vivo* following endothelial cell loss or damage highly inefficient.

When a foreign cell is transplanted into a host, the immune system of the host rapidly mobilizes to destroy the cell and thereby protect the host. The immune system attack on the foreign cell is referred to as transplant rejection. The organism's first line of defense is through either lyric destruction or the activation of procoagulant and prothrombotic properties of the donor endothelial cell that may result from activation of the host's complement system and is generally known as the "hyperacute rejection response" or simply the "hyperacute response."

Several studies have demonstrated that the hyperacute response to transplants of either xenogeneic (from different species) and allotypic (from different individuals of the same species) organs is mediated by antibody-dependent activation of the complement system at the surface of the donor endothelium, as discussed, for example, by Platt et al. , 1990 "Transplantation of discordant xenografts: a review of progress" Immunology Today 11:450-456. That is, the complement system attacks the endothelial cells lining the vessels of the transplanted organ.

The complement system is a complex interaction of plasma proteins and membrane cofactors which act in a multistep, multi-protein cascade sequence in conjunction with other immunological systems of the host organism. The classic complement pathway involves an initial antibody recognition of, and binding to, an antigenic site on a target cell. This surface bound antibody subsequently reacts with the first component of complement, Clq, forming a Cl-antibody complex with Ca2+, Clr, and Cls which is proteolyticaity active. Cls cleaves C2 and C4 into active components, C2a and C4a. The C4b,2a complex is an active protease called C3 convertase, and acts to cleave C3 into C3a and C3b. C3b forns a complex with C4b,2a to produce C4b,2a,3b, which cleaves C5 into C5a and C5b. C5b forms a complex with C6 and this complex interacts with C7 in the fluid phase thereby exposing hydrophobic domains within C5b and C6 that stabilize the C5b,6,7 ternary complex in the cell membrane. C8, which is in the fluid phase, then binds to the C5b, 6, 7 ternary complex and this complex may contribute to the development of functional membrane lesions and slow cell lysis. Upon binding of C9 to C8 in the C5b-8 membrane complex, lysis of foreign cells is rapidly accelerated.

Copending U.S. patent application serial number 07/365,199, filed June 12, 1989, allowed, assigned to the Oklahoma Medical Research Foundation discloses that the human complement regulatory protein CD59 can be used to protect non-human endothelial cells, for example, porcine endothelial cells, from attack by human complement, either when provided in solution with the cells or expressed in genetically engineered cells. See also Zhao et al., 1991 "Amplified gene expression in CD59-transfected Chinese Hamster Ovary cells confers protection against the membrane attack complex of human complement" J. Biol. Chem. 266:13418-13422. The homologous complement inhibitory activity of CD59 resides in its species-specific interaction with the terminal complement components C8 and C9, as further reported by Rollins and Sims, 1990 "The complement inhibitory activity of CD59 resides in its capacity to block incorporation of C9 into membrane C5b-9" J. Immunol. 144:3478-3483.

Although the use of CD59 does successfully address the problem of hyperacute rejection as a result of complement attack, it does not protect the cell against the overall immune attack of the host organism against foreign endothelial cells.

In stimulating immune responses, antigens elicit many molecular and cellular changes. Lymphocytes recognize antigens as foreign and are responsible for initiating both cellular and humoral responses against the presenting antigen. B lymphocyte cells respond to antigen by the production of antibodies against the presenting antigen; T lymphocytes respond by initiating a cellular response to the presenting antigen. The two major subsets of T cells are T_{H} cells, involved in processing of antigen for presentation to B cells, characterized by the presence of a cell-surface glycoprotein called CD4, and cytolytic T lymphocytes (CTLs), involved in recognition of antigen on cell surfaces and lysis of cells recognized as foreign, characterized by the presence of a cell-surface glycoprotein called CD8. T cells recognize peptide fragments in conjunction with one of the two main classes of cell-surface glycoproteins of the major histocompatibility complex (MHC): either class I (MHC-I) or class II (MHC-II) proteins. CD8+ T cells recognize antigens in conjunction with MHC-I, whereas CD4+ T cells recognize them in conjunction with MHC-II.

The MHC contain three major classes of genes. Class I genes encode the principal subunits of MHC-I glycoproteins, called human leukocyte antigens in humans, the principle ones being HLA-A, B, and C. These are present on virtually all cells and play a major role in rejection of allografts. They also form complexes with peptide fragments of viral antigens on virus-infected cells: recognition of the complexes by CD8+ CTLs results in destruction of virus infected cells. Recognition of the complexes is by a single receptor on the T cells which recognizes antigen in combination with MHC.

Class II genes, the major classes in humans being known as DP, DQ (subclasses β2, α2, and β1α1) and DR (subclasses β1, β2, β3 and α1), encode cell-surface glycoproteins that are expressed by antigen-presenting cells, principally B cells, macrophages and dendritic cells. Together with peptide fragments of antigens, the class II proteins form the epitopes that are recognized by T helper cells (CD4+). Class III genes encode at least three proteins of the complement cascade and two cytotoxic proteins, tissue necrosis factor and lymphotoxin, which are involved in diverse immune reactions that destroy cells.

T-cell mediated immune reactions can be organized into three sequential activation steps. First, CD4+ and CD8+ T lymphocytes (T-cells) recognize the presence of non-autologous MHC class II and class I proteins, respectively, on the surface of the foreign cell.

Second, the T-cells are activated by interaction of a ligand with the T cell receptors and other accessory stimulatory molecules, so that activation depends upon a variety of variables including humoral signals such as cytokines received by protein receptors on the surface of the cells. Most important is the interaction between the antigen specific T cell receptor and ligand, a complex of MHC and antigenic peptide on the antigen presenting cell (APC). Other receptors present on the T cell must also be contacted by their ligands on APC to insure activation. Once activated, the T-cells synthesize and secrete interleukin-2 (IL-2) and other cytokines.

The cytokines secreted by the activated T-cells lead to the third, or effector, phase of the immune response which involves recruitment and activation of lymphocytes, monocytes, and other leukocytes which together lead to cell lysis, as reviewed, for example, by Pober et al., 1990 "The potential roles of vascular endothelium in immune reactions" Human Immunol. 28:258-262.

Historically, attempts to interrupt the T-cell immune response have generally met with limited success. For example, several strategies have tried to use reagents of various types, including antibodies and blocking proteins, to interfere with adhesion between T-cells and foreign cells. Lider et al., 1988 "Anti-idiotypic network induced by T cell vaccination against experimental autoimmune encephalomyelitis" Science 239:181 reported on the use of T-cell vaccines; Owhashiand et al., 1988 "Protection from experimental allergic encephalomyelitis conferred by a monoclonal antibody directed against a shared idiotype on rat T cell receptors specific for myelin basic protein" J. Exp. Med, 168:2153, reported on the use of T-cell receptor blocking antibodies; Brostoffand et al. 1984 ''Experimental allergic encephalomyelitis: successful treatment *in vivo* with a monoclonal antibody that recognizes T helper cells" J. Immunol. 133:1938 reported on the use of antibodies to CD4; and Adorini et al., 1988 "Dissociation of phosphoinositide hydrolysis and Ca2+ fluxes from the biological responses of a T-cell hybridoma" Nature 334:623-628, reported on the use of blocking peptides that occupy T-cell receptors. These strategies have generally resulted in immune responses to the reagents, rather than the desired interruption of T-cell binding.

It would clearly be advantageous if one could decrease the probability of T-cell mediated reaction against transplanted cells, as well as complement-mediated attack and lysis of the cell.

It is therefore an object of the present invention to provide an improved method and compositions for constructing endothelial cells that are resistant to both complement and cellular attack when transplanted into a foreign host.

It is a further object of the present invention to provide genetically engineered cells that are not recognized as foreign when implanted into a foreign host and therefore evade attack by the immune system.

It is still further object of this invention to provide genetically engineered cells which after transplantation can resist complement-mediated attack and evade lymphocyte-mediated lysis, specifically CD4 + T-lymphocytes, and preferably CD8 + T-lymphocytes.

It is another object of the invention to provide a mechanism for selectively killing such genetically engineered cells when their presence in the host is no longer desired.

It is still another object of the present invention to provide a biological vehicle for delivery of therapeutic products.

### Summary of Invention

In accordance with the invention there is provided a genetically engineered mammalian cell for transplantation into a human or animal
wherein the cell does not express on its surface proteins encoded by either the class I major histocompatibility complex genes or the class II major histocompatability complex genes which elicit a T lymphocyte mediated reaction against the cell and
the cell expresses a stably incorporated nucleotide molecule which codes for a protein inhibiting complement mediated attack of the engineered cell when introduced into an animal of another species or another individual.

In particular, genetically engineered cells are provided which include a DNA sequence which is expressed by the cell and which codes for a protein having complement inhibitory activity that is not normally expressed in the cell. These cells may also be engineered so that they do not express on their cell surfaces functional proteins encoded by the class II major histocompatibility complex (MHC) genes, the HLA DP, DQ, and DR genes in human cells, or their equivalent in cells of a different species. Further, the genetically engineered cells may not express on their cell surfaces the proteins encoded by the class I MHC genes, the HLA A, B and C genes in human cells, or their equivalent in cells of a different species. In some embodiments, the cells include a genetic (DNA) sequence which is expressed by the cell and which codes for a protein which in the presence of a selected agent results in death of the cell.

The genetic sequence which codes for a protein which has complement regulatory activity protects the cell from hyperacute rejection through attack and lysis resulting from activation of the complement system. The removal of the cell surface proteins encoded by the class I (for example, HLA A, B and C) and class II (for example, HLA DP, DQ, and DR) MHC genes makes the cells substantially unrecognizable by the host's CD8+ and CD4+ T-lymphocytes, respectively. The genetic sequence which codes for a protein which can produce cell death provides a mechanism for eliminating the genetically engineered cells from the host when their presence is no longer desired.

The cells are modified in culture using standard *in vitro* transfection techniques, or can be derived from transgenic mice modified as embryos. These modified cells can serve as universal donor cells for administering therapeutic agents to the host or as replacements for natural cells which have been damaged or lost. In the most preferred embodiment, the cells are dissociated endothelial cells.

### Brief Description of the Drawings

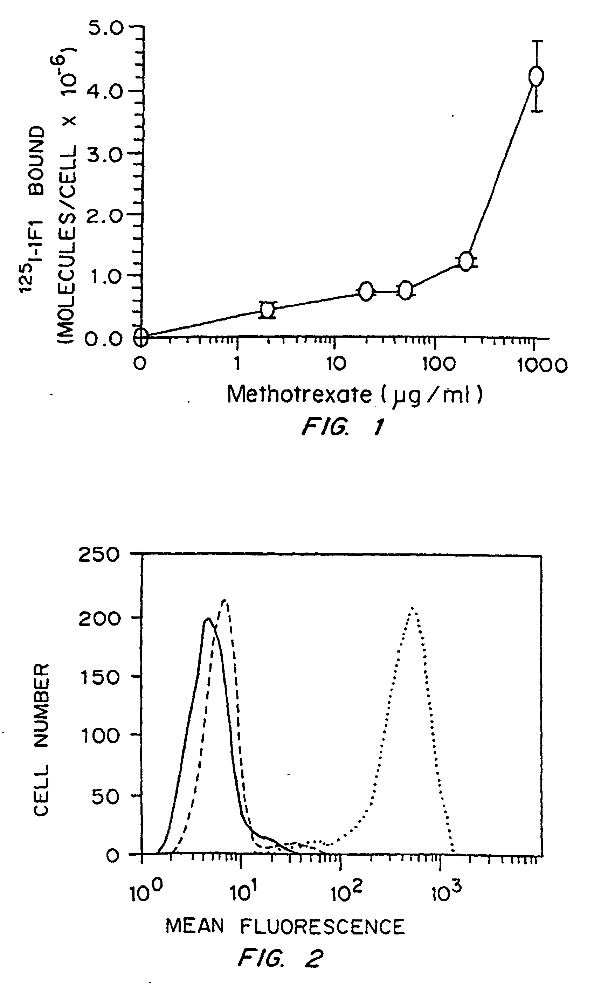

Figure 1 is a graph of the induction of CD59 antigen in CHO cells transfected with plasmid containing human CD59 cDNA, ¹²⁵I-1F1 bound CD59 (molecules/cell x 10⁻⁶) versus methotrexate (µg/ml). Chinese hamster ovary cells were transfected with a plasmid containing the pFRSV vector and cDNA for human CD59. After subcloning and selection, the cells were maintained in medium containing methotrexate and surface antigen measured by the specific binding of monoclonal antibody ¹²⁵I-1F1 (10 µg/ml) against CD59. All data were corrected for nonspecific binding measured for control (nontransfected) CHO cells grown in the absence of methotrexate (origin). Data denote means ± S.E. of three measurements made on separate days.
Figure 2 is a graph of the removal of cell-surface CD59 by phosphatidylinositol-specific phospholipase C (PIPLC), plotting cell number versus mean fluorescence. CD59-transfected CHO cells amplified by growth in 1 mg/ml methotrexate were suspended at 2 x 10⁶/ml in HBSS and incubated for 1 h at 37°C with either 0 (....) or 1 (---) unit/ml phosphatidylinositol-specific phospholipase C. Cell-surface CD59 was then measured by flow cytometry using monoclonal antibody 1F1 (10 µg/ml), which was detected with FITC anti-mouse IgG (67 µg/ml). Histograms denote mean fluorescence per cell on logarithmic scales. Also shown is background cell fluorescence measured in the absence of 1F1 (---).

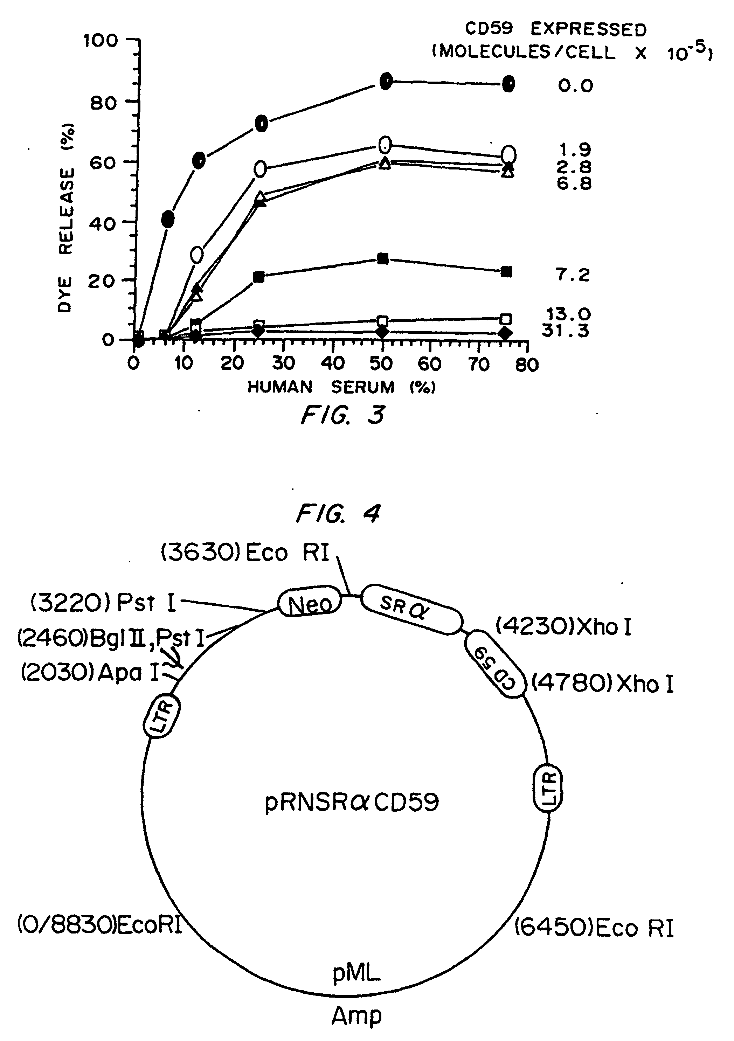

Figure 3 is a graph showing protection of CD59-transfected CHO cells from human serum complement, dye release (%) versus human serum (%). CD59-transfected CHO cells were induced to express various amounts of CD59 antigen by growth in methotrexate-containing media, molecules CD59 expressed/cell x 10⁻⁵: 0.0 (dark circles), 1.9 (open circles), 2.8 (dark diamonds), 6.8 (open triangles), 7.2 (dark squares), 13.0 (open squares), and 31.3 (dark diamonds).
Figure 4 shows the structure of the retroviral vector used in Example 2. This vector was constructed from a defective Moloney murine leukemia virus. The SV40 promoter was excised and replaced with the SRalpha promoter. A 500 bp cDNA fragment containing the CD59 coding sequence was cloned into an *Xho*I site and verified by restriction analysis. The resulting plasmid was designated pRNSRαCD59. Ecotropic retrovirus was produced by transfecting Psi-2 cells with polybrene and selecting in the toxic aminoglycoside G418. Amphotropic virus stocks were prepared by infecting the amphotropic packaging cell line Psi-AM with the ecotropic virus, were added directly to endothelial cell cultures in the presence of polybrene, and transfectants were selected with 400 µg/ml G418.

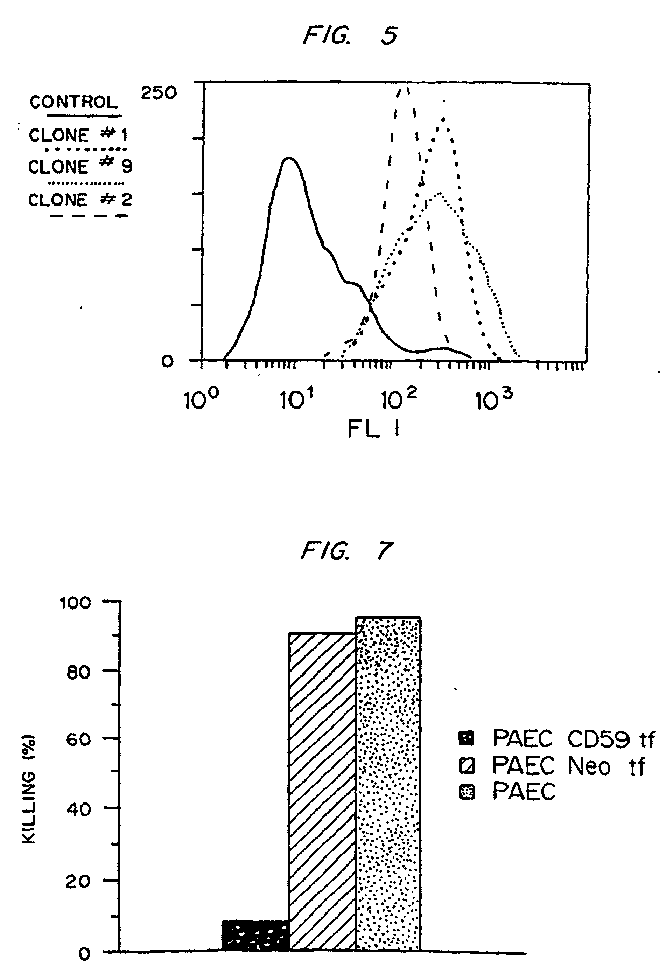

Figure 5 is a graph of cell surface expression of human CD59 on porcine aortic endothelial cells (PAEC) as detected by anti-CD59 antibody and analyzed by flow cytometric analysis. The solid line represents the fluorescence intensity of PAEC infected with retrovirus shown in Figure 4 carrying only the control neomycin resistance gene. The dashed line, small dotted line, and larger dotted line represent the fluorescence intensity of CD59-expressing PAEC cell clones 2, 9, and 1, respectively.

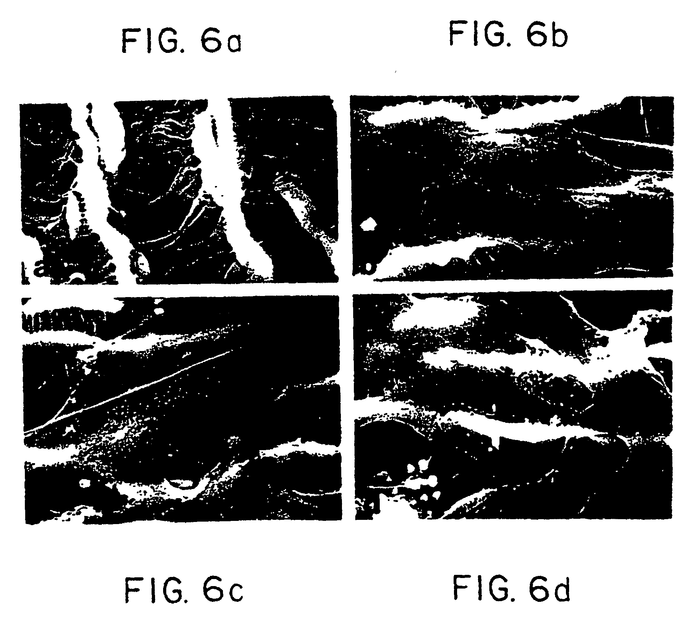

Figure 6 shows a scanning electron micrograph of CD59-expressing PAEC attached to a synthetic Gortex™ graft. Figure 6a is the control Gortex™, Figures 6b, c, and d are Gortex™ with CD59-expressing cells implanted thereon.

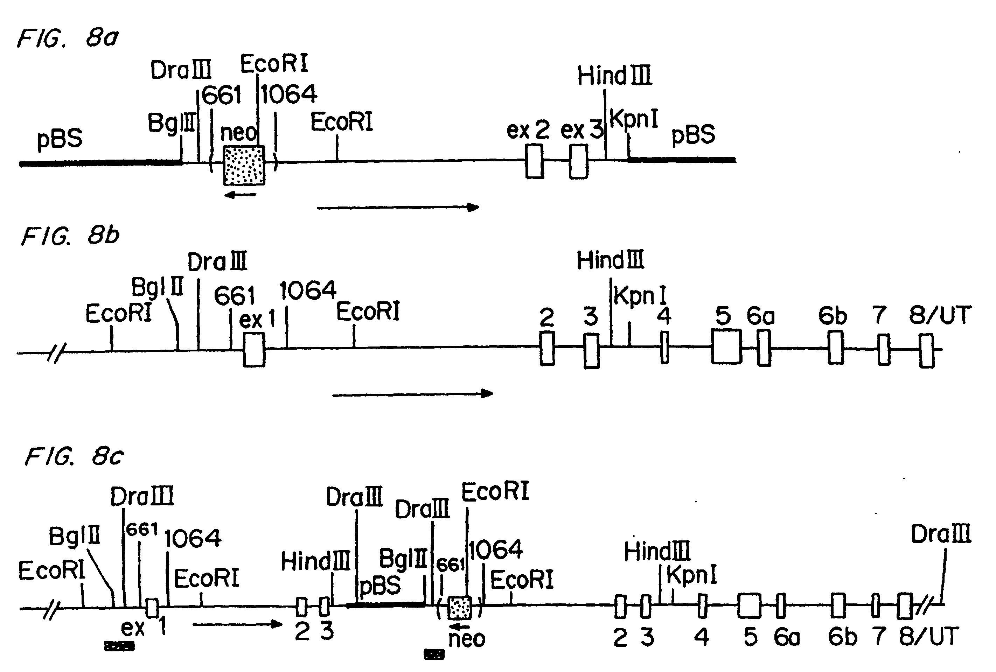

Figure 7 is a bar graph showing the protection of human CD59-expressing PAEC from lysis by human complement. The solid bar represents the percentage of cell lysis of PAEC expressing human CD59. The cross-hatched bar represents the percentage of cell lysis of PAEC expressing only the control neomycin resistance gene while the stippled bar represents the percentage of cell lysis of control (noninfected) PAEC.

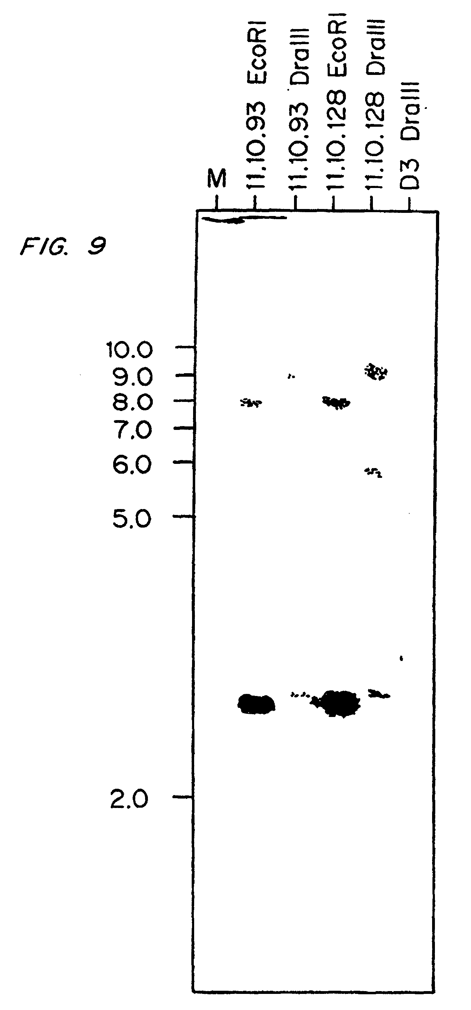

Figure 8A shows a restriction digest map of the gene targeting vector for the mouse invariant chain gene cloned into pBS (Bluescript). The targeting vector contains the neomycin gene (neo). Figure 8B shows a partial restriction digest map of the endogenous mouse invariant chain gene and Figure 8C shows a restriction digest map of the disrupted invariant chain gene achieved by homologous recombination. Arrows indicate the direction of transcription in all three panels. The recognition region for the radiolabeled invariant gene probe used for the Southern blot shown in Figure 9 is indicated by a solid bar below Figure 8C.
Figure 9 is a Southern blot showing the restriction digestion pattern for two independent neomycin resistant mouse embryonic stem cell clones where the endogenous invariant chain gene has been disrupted by homologous recombination and replaced with a mutated form of the gene. The two clones are designated 11.10.93 and 11.10.128. Size markers are indicated on the left side. The *Dra*III restriction pattern of the parental cells is indicated in the far light lane and is clearly different from the restriction pattern of the two clones carrying the modified invariant chain gene.

### Detailed Description of the Invention

### I. Protection From Hyperacute Rejection

The lysis of cells by complement has been determined to typically require only the terminal complement components, in contrast to previous reports that it may be essential to interrupt complement activation at the C3 stage, as described, for example, in PCT application WO 91/05855.

Sequential addition of C6,7,8, and 9 to C5b leads to the formation of a membrane attack complex (MAC), a pore-like complex which, when inserted into the plasma membrane of the target cell, increases membrane permeability to calcium and other ions. Consequently, lysis of the plasma membrane ensues and the cell is either destroyed, or alternatively, there is a non-lytic alteration of specific cell functions affecting vascular hemostasis. In the case of human endothelial cells exposed to human serum complement, membrane deposition of the C5b-9 complex initiates a variety of procoagulant and prothrombotic changes in the cell that are expected to accelerate blood clotting and thrombus formation, as described, for example, by Hattori, et al., 1989 "Complement proteins C5b-9 induce secretion of high molecular weight multimers of endothelial von Willebrand Factor and translocation of granule membrane protein GMP-140 to the cell surface" J. Biol. Chem. 264:9053-9060; Hamilton, et al., 1990 "Regulatory control of the terminal complement proteins at the surface of human endothelial cells: Neutralization of a C5b-9 inhibitor by antibody to CD59" Blood 76:2572-2577; and Hamilton and Sims 1991 "The terminal complement proteins C5b-9 augment binding of high density lipoprotein and its apoproteins A-I and A-II to human endothelial cells" J. Clin. Invest. 88:1833-1840. These responses appear to depend upon insertion of C9 into the plasma membrane of the target cell and therefore can be prevented by interfering with assembly of the C5b-9 complex.

### Membrane proteins inhibiting complement.

Specific membrane proteins which exhibit potent inhibitory activity for the complement cascade have been isolated and molecularly cloned.

In particular, with regard to the human complement system, protection against the pore-forming activity of the C5b-9 complex can be conferred on non-primate cells by txansfection of such cells with a cDNA encoding the human complement regulatory protein CD59.

The capacity to stably express CD59 in Chinese hamster ovary (CHO) cells has enabled direct evaluation of the C5b-9 inhibitory activity conferred when CD59 is selectively expressed in mammalian cells that normally express neither CD59 nor HRF. The results demonstrate that the inhibitory activity of human blood cells toward the membrane attack complex of human serum complement can be transferred to a non-human mammalian cell by transfection with the CD59 cDNA and demonstrate that the C5b-9 inhibitory function of this protein correlates with the amount of newly expressed surface CD59 antigen.

The existence of these proteins and the studies detailed below indicate that a deletion or inactivation of these cell surface components increases the risk of vascular thrombosis and lead to a decreased storage time for platelets and platelet rich plasma (PRP), and perfused organs and transplanted tissue. Accordingly, the survival and hemostatic efficacy of platelets, the survival and function of hematopoietic progenitor cells, such as CFU-S, CFU-GEMM, and CFU-L, and their progeny, such as BFU-E, BFU-MK, and CFU-GM, as well as the mature blood cells, including erythrocytes, platelets, monocytes, granulocytes, and lymphocytes, that may derive from these progenitor cells after bone marrow transplantation, as well as the survival of organs and tissue for transplant, which are collected and stored *in vitro*, can be increased by addition of the C5b-9 inhibitor to the storage buffer or perfusate and/or by the introduction and expression of the gene encoding CD59 in the cells to be protected. Autoimmune disorders and other disease states that involve C5b-9 mediated platelet activation, including lupus, rheumatoid arthritis, and additional types of immuno-vasculitis, can also be treated by the intravascular administration and/or transfection and expression of an effective amount of the inhibitor or a functionally active polypeptide thereof to suppress C5b-9 activity in a patient requiring such treatment. Similar uses of the inhibitor can be applicable for cell culture in human blood derived culture media.

The data shown in the examples below are evidence that transfection with the gene for CD59 can be used to confer protection against the membrane attack complex of complement to cells that do not normally restrict activation of the human C5b-9 proteins. These data confirm by DNA transfection the C5b-9 inhibitory function that has previously been attributed to CD59 antigen present on human erythrocytes and exclude the possibility that the activity found associated with this protein reflects the presence of another membrane constituent with complement inhibitory activity that copurifies with CD59 antigen. Despite apparent differences in glycosylation, the C5b-9 inhibitory function observed for recombinant CD59 expressed in CHO cells exhibits specificity for human C8 and C9 (within C5b-9), analogous to that observed for the human erythrocyte membrane and for purified erythrocyte CD59 antigen. This capacity to confer species-selective protection against the human C5b-9 proteins by transfection of a non-human cell with cDNA encoding the CD59 sequence establishes unequivocally that this 18-21 kD protein functions as a homologous complement restriction factor on human blood cells and is consistent with the observation that the syndrome of paroxysmal nocturnal hemoglobinuria can be associated with an isolated deficiency of erythrocyte CD59.

As illustrated by the following examples, the complement inhibitory activity of recombinant CD59 was found to saturate when the expression of surface antigen was amplified to greater than or equal to 1.3 X 10⁶ molecules/CHO cell. Assuming a spherical diameter of approximately 25 µm for the CHO cell, this is equivalent to greater than or equal to 600 molecules of CD59 antigen/µm² of plasma membrane surface. By comparison, human erythrocytes, which are highly resistant to activation and lysis by human complement, express approximately 2.5 X 10⁴ molecules of CD59 antigen/cell, which is equivalent to approximately 200 molecules/µm² of membrane surface. Extrapolating from this data, 1 x 10³ molecules CD59/cell or greater than or equal to 1 molecule of CD59 antigen/µm² of plasma membrane surface should be effective in inhibiting complement mediated activation and lysis.

The data also demonstrate that recombinant CD59 expressed in CHO cells exhibits the species-selective recognition of human C5b-9 characteristic of CD59 in human erythrocytes despite apparent differences in *N*-linked glycosylation. These data indicate that the species selectivity exhibited by CD59, which includes recognition for human C8 (within C5b-8) and human C9 (within C5b-9), is conferred by the core protein, independent of its carbohydrate, or that the relevant carbohydrate structures are conserved in the recombinant protein when expressed in CHO cells. As used herein in the compositions and methods for the prolongation of platelet and organ survival and enhancement of therapeutic efficacy or suppression of complement mediated disorders, "C5b-9 inactivator" refers to any CD59 molecule, including the 18 kDa protein on erythrocyte membranes, peptide fragments thereof having C5b-9 inhibitory activity, preferably containing a membrane binding domain, whether isolated from naturally produced materials or recombinantly engineered sequences. The term also includes cells infected or transfected with, and expressing, the gene for CD59 or a biologically functional portion thereof, as well as cells in transgenic mice in which the gene in combination with a promoter such as the murine K^{d} MHC class I promoter has been stably introduced into an embryo of the animal using a technique such as microinjection. All molecular weights are determined by SDS-PAGE under non-reducing conditions.

Other complement inhibitors which have been identified and can be used alone or in combination with CD59 include:
(1) CD46, also known as membrane cofactor protein (MCP), as described by Purcell, et al., 1990 "The human cell surface glycoproteins HuLy-m5, membrane cofactor protein (MCP) of the complement system, and trophoblast leucocyte common (TLX) antigen, are CD46" J. Immunol, 70:155-161; and Seya and Atkinson, 1989 "Functional properties of membrane cofactor protein of complement" Biochem. J. 264:581-588. This inhibitor functions by binding to complement component C3b thereby activating molecules that cleave C3b into inactive fragments preventing accumulation of C3b and, therefore, its contribution to the formation of the MAC. See also White et al. 1991 "Protection of mammalian cells from human complement-mediated lysis by transfection of human membrane cofactor protein (MCP) or decay accelerating factor (DAF)" Int. Meeting on Xenotransplantation -: - - - (recombinant human CD46 shown to provide protection of non-primate cells from lysis by human complement).
(2) CD55, also known as decay accelerating factor (DAF), described by Nicholson-Weller et al., 1982 "Isolation of a human erythrocyte membrane glycoprotein with decay-accelerating activity for C3 convertases of the complement system" J. Immunol. 129:184; Lublin and Atkinson, 1989 "Decay accelerating factor: Biochemistry, molecular biology, and function" Annu. Rev. Immunol. 7:35; Lublin et al., 1987 "The gene encoding decay-accelerating factor (DAF) is located in the complement-regulatory locus on the long arm of chromosome 1" J. Exp. Med. 165:1731; and Medof et al., 1987 "Cloning and characterization of cDNAs encoding the complete sequence of decay accelerating factor of human complement" Proc. Natl. Acad. Sci. USA 84:2007. This inhibitor is a membrane bound protein of approximately 70 kD in molecular mass which interferes with the assembly of C3 convertase. See also White et al., 1991, reporting that recombinant DAF provides protection of non-primate cells from lysis by human complement.

The relative contributions of CD46, CD55, and CD59 in providing protection from complement-mediated lysis has been assessed in human amnionc epithelial cells (HAEC) by the use of specific blocking antibodies, as reported by Rooney et al., 1990 "Protection of human amniotic epithelial cells (HAEC) from complement-mediated lysis: expression on the cells of three complement inhibitory membrane proteins." Immunology 71:308-311. The results demonstrated that CD59 provides the most protection against complement attack, as compared with CD46 and CD55. Additionally, a patient with paroxysmal nocturnal hemoglobinuria, a rare disorder caused by an unusual susceptibility of erythrocytes to the lytic action of complement, was described as having an inherited deficiency of CD59 without a deficiency of CD55, by Yamashina et al. 1990 "Inherited complete deficiency of 20-kilodalton (CD59) as a cause of paroxysmal nocturnal hemoglobinuria" New Engl. J. Med. 323:1184-1189.

By contrast to the intravascular hemolysis observed for this patient reported to be deficient in CD59 but normal for decay accelerating factor (and presumably normal for other complement inhibitors), individuals with inherited defects or deficiencies in erythrocyte CD55 (Decay Accelerating Factor) generally do not exhibit intravascular hemolysis, as reported by Daniels, G. 1989 "Cromer-related antigens-blood group determinants on decay accelerating factor. Vox. Sang. 56:205; Holguin, et al. 1992 "Analysis of the effects of activation of the alternative pathway of complement on erythrocytes with an isolated deficiency of decay accelerating factor. J. Immunol. 148:498-502, suggesting the CD59 is necessary and sufficient to protect these cells from the cytolytic effects of complement in human plasma.

Cells suitable for transplantation into a foreign host are protected from complement-mediated lysis by introducing into the cell DNA encoding a protein, or combination of proteins, inhibiting complement-mediated lysis, for example, CD59, CD55, CD46 and/or other inhibitors of C8 or C9. CD59 is the preferred inhibitor, introduced into the cells by transfection or infection with a vector encoding the CD59 protein, and expressed on the surface of the transfected/infected cells. The inhibitor is preferably of the same species of origin as the host into which the cells are to be transplanted.

The gene encoding the complement inhibitor can be introduced into a cell of a different species of origin, for example, a human CD59 gene can be introduced into a porcine cell so that the cell resists attack when transplanted into a human, or the gene can be introduced into a cell of the same species of origin so that increased amounts of the protein are expressed on the surface of the cell. For example, the gene can be placed under the control of a promoter enhancing expression of the gene which is then inserted by homologous recombination into the host cell chromosome at the site where the gene is normally located, but under the control of the promoter which enhances expression, or can be inserted into the chromosome at another locus on the chromosome.

DNA sequence information for CD46, CDS5 and CD59 has been reported in the literature.

The sequence reported by Lublin et al., 1988 "Molecular cloning and chromosomal localization of human membrane cofactor protein (MCP): Evidence for inclusion in the multi-gene family of complement-regulatory proteins" J. Exp. Med. 168:181-194, for CD46 is shown below (Sequence I.D. No. 1).

The sequence reported by Medof et al., 1987, for CD55 is shown below (Sequence I.D. No. 2). **BOLD TEXT** = HUMDAFC1 (Promoter and 5' end of Exon 1, genomic Sequence)
PLAIN TEXT = HUMDAF cDNA

The amino acid and nucleic acid sequences reported by Philbrick, W.M., et al., 1990 Eur. J. Immunol. 20, 87-92, for CD59 are as follows (Sequence I.D. No. 3).

The amino acid sequence for the protein is:

A cDNA sequence encoding the CD59 protein is (Sequence I.D. No. 4):

Matching oligonucleotide primers can be readily designed and then used to obtain full length cDNA sequences for these proteins by performing a polymerase chain reaction amplification on human cDNA. The oligonucleotide primers are preferably designed with specific restriction enzyme sites so that the full length cDNA sequences can be readily subcloned into vectors for use in transfecting/infecting the target donor cells.

### Introduction of DNA encoding the Complement Inhibitors into the Endothelial Cells.

DNA encoding the complement inhibitors can be introduced into the cells in culture using transfection or into embryos for production of transgenic animals expressing the complement inhibitors on the surface of their cells.

### Introduction into cells in culture.

As known in the art, transfection can be accomplished by electroporation, calcium phosphate precipitation, a lipofectin-based procedure, or micromjection or through use of a "gene gun". In each case, CDNA for the inhibitory protein, such as CD59, is subcloned into a plasmid-based vector which encodes elements for efficient expression in the genetically engineered, cell. The plasmid-based vector preferably contains a marker such as the neomycin gene for selection of stable transfectants with the cytotoxic aminoglycoside G418 in eukaryotic cells and an ampicillin gene for plasmid selection in bacteria.

Infection, which for endothelial cells is preferred, is accomplished by incorporating the genetic sequence for the inhibitory protein into a retroviral vector. Various procedures are known in the art for such incorporation. One such procedure which has been widely used in the art employs a defective murine retrovirus, Psi-2 cells for packaging the retrovirus, and the amphotropic packaging cell line Psi-AM to prepare infectious amphotropic virus for use in infecting the target donor cells, as described by Kohn et al., 1987 "Retroviral-mediated gene transfer into mammalian cells" Blood Cells 13:285-298.

Alternatively, rather than a defective Moloney murine retrovirus, a retrovirus of the self-inactivating and double-copy type can be used, such as that described by Hantzopoulos et al., 1989 "Improved gene expression upon transfer of the adenosine deaminase minigene outside the transcriptional unit of a retroviral vector" Proc. Natl. Acad. Sci. USA 86:3519-3523.

### Introduction into Embryos for production of transgenic mice expressing complement inhibitor on the surface of their cells.

A variety of methods are known to those skilled in the art for making transgenic animals expressing a complement inhibitory protein on the surface of the cells for use as a source of modified cells for transplantation. Examples of particularly useful animals include transgenic mice. The most well known method for making a transgenic mice is by superovulation of a donor female, surgical removal of the egg and injection of the genetic material in the pronuclei of the embryo, as taught by U.S. Patent No. 4,873,191 to Wagner, the teachings of which are incorporated herein. Another commonly used technique involves the genetic manipulation of embryonic stem cells (ES cells), as specifically described below in Example 3.

ES cells are grown as described, for example, in Robertson, E.J. "Embryo-derived stem cell lines" in: Teratocarcinomas and embryonic stem cells: A practical approach. E.J. Robertson, ed. 71-112 (Oxford-Washington, D.C.: IRL Press, 1987). Genetic material is introduced into the embryonic stem cells, for example, by electroporation according to the method of McMahon, A.P;, and Bradley, A. Cell 62, 1073-1085 (1991). Colonies are picked from day 6 to day 9 of selection into 96 or 24 well dishes (Costar) and expanded and used to isolate DNA for Southern blot analysis.

Chimeric mice are generated as described in Bradley, "Production and analysis of chimaeric mice" in Teratocarcinomas and embryonic stem cells: A practical approach E.J. Robertson, ed. pp. 113-151 (Oxford, Washington, D.C. IRL Press 1987), the teachings of which are incorporated herein. Genetic material is injected into blastocysts. From those implanted females that become pregnant, chimaeras are selected from the offspring and bred to produce germline chimaeras for use as donor animals.

### II. Protection From T-Cells

In contrast to the previous efforts to block the T cell immune-mediated response using antibodies or blocking compounds, genetic engineering of the cells are used to interrupt the T-cell immune response. The donor endothelial cells are genetically engineered to not express on their surface proteins encoded by either the class I major histocompatibility complex genes or the class II major histocompatability complex genes which elicit a T-lymphocyte mediated reaction against the cell. More preferably, the cells are engineered to not express substantially all cell surface class I and class II MHC molecules. As used herein, the term "not express" may mean either that an insufficient amount is expressed on the surface of the cell to elicit a response or that the protein that is expressed is deficient and therefore does not elicit a response.

As used herein, the MHC molecules are referred to as HLA molecules, specifically of classes HLA A, B and C, and class II HLA DP, DQ, and DR, and their subclasses. This terminology is generally construed as specific to the human MHC, but is intended herein to include the equivalent MHC genes from the donor cell species, for example, if the cells are of porcine origin, the term HLA would refer to the equivalent porcine MHC molecules, whether MHC I or II.

When the class II MHC molecules are removed, CD4+ T - cells do not recognize the genetically engineered endothelial cells; when both the class I and class II MHC molecules are removed neither CD4+ nor CD8+ cells recognize the modified cells.

The relative importance of the CD4+ and CD8+ T-cell subpopulations in mediating immune responses, in particular allograft rejection, has been approached experimentally. Both experiments of nature and gene targeting by homologous recombination have provided some insights. For example, the AIDS virus (HIV) selectively depletes CD4+ T-cells and not CD8+ T-cells and virtually destroys the body's immune defense. Additionally, although homologous recombination and disruption of the β2-microglobulin gene in mice results in elimination of CD8+ T-cells, the mice inheriting this genotype remain healthy and are capable of resisting infection by foreign organisms such as viruses, as reported by Zijlstra et al., 1989 "Germ-line transmission of a disrupted β2-microglobulin gene produced by homologous recombination in embryonic stem cells" Nature 342:435438; and Koller et al., 1990 "Normal development of mice deficient in β2M, MHC class I proteins, and CD8+ T cells" Science 248:1227-1230. These two observations together suggest that CD4+ T-cells play a central and essential role in immune responses in general, while CD8+ T-cells play a specialized and less essential role in host defense mechanisms.

The preferred genetic modification performed on the endothelial cells includes 1) disrupting the endogenous invariant chain gene which functions in the assembly and transport of class II MHC molecules to the cell surface and loading of antigenic peptide, and 2) disrupting the endogenous β₂-microglobulin gene (β₂M gene) which codes for a protein required for the cell surface expression of all class I MHC molecules. Invariant chain is believed to be required for the insertion of andgienic peptide fragments into the MHC class II molecule. Together, the antigenic peptide and MHC is recognized by T cells. In the absence of antigenic peptide, T cell recognition is not normally obtained, nor is the MHC class II molecule folded properly. Thus, in cells lacking invariant chain, presentation of peptide will be abrogated and even if minuscule amounts of cell surface MHC are obtained, they may be devoid of peptide and therefore, non-immunogenic.

The disruption of these genes is accomplished by means of a homologous recombination gene targeting technique, as described by Zijlstra et al., 1989; Koller et al., 1990; and Example 2 below showing disruption of the invariant chain gene.

The technique is applied to suppress expression of the class I MHC proteins on the cell surface as follows. First, the complete β₂M gene for the target donor endothelial cell is cloned, e.g., for porcine endothelial cells the porcine β2M gene is cloned. This is done by first obtaining cDNA for a homologous β₂M gene, such as the mouse β2M gene. DNA sequence information for the mouse β₂M cDNA has been reported by Panics et al., 1983 Nature 302:449-452. Matching oligonucleotide primers are readily designed to hybridize by complementary base pairing to the extreme 5' and 3' ends of the mouse β₂M cDNA. These oligonucleotide primers are then used to obtain full-length cDNA sequences for the mouse β₂M protein by performing a polymerase chain reaction amplification on mouse cDNA. The oligonucleotide primers are preferentially designed to encode specific restriction sites at their ends so that full-length cDNA sequences can be readily subcloned into plasmids.

The full-length mouse β₂M cDNA can then be used as a radiolabeled hybridization probe to screen cDNA libraries prepared from the source of the target donor endothelial cells, e.g., for porcine endothelial cells the mouse β₂M cDNA is used as a hybridization probe to screen a porcine cDNA library which has been cloned into a lambda phage vector. Positive hybridizing clones are selected, purified, subcloned into plasmid vectors and then sequenced using methods known in the art.

The complete porcine β₂M gene, including untranslated nucleotide residues as well as the portion of the gene which codes for the expressed protein, can then be cloned by screening a porcine genomic DNA library cloned into a lambda phage vector with radiolabeled porcine β₂M cDNA as a hybridization probe. Positive clones are selected, purified, subcloned into plasmid vectors and sequenced using methods known in the art.

Once cloned, the β₂M gene is subcloned into a plasmidbased or preferentially a retroviral-based vector (the "gene targeting vector") such that the reading frame of the β₂M gene is disrupted by insertion of a short DNA sequence which allows for positive selection of recombination in the endothelial cells, for example, a neomycin resistance gene (hereinafter referred to as the "positive selection gene"). The gene targeting vector also carries an additional selection gene (the "negative selection gene"), outside of the disrupted β₂m gene region which allows for selection against non-homologous recombination, i.e., for selection against incorporation of the entire plasmid into the genetic information of the cell rather than just the portion of the plasmid carrying the disrupted β₂M gene. The negative selection gene can be, for example, a herpes simplex thymidine kinase gene.

The gene targeting vector is then transfected/infected into the cells as described above and homologous recombination events are selected by screening for clones which express the positive selection gene but not the negative selection gene.

The same procedures are used to achieve homologous recombination of the invariant chain gene as demonstrated in Example 3 below.

### III. Cells to be Treated. Engineered and Hosts

### A. Genetic Engineering of Cells to protect from complement- and T cell- mediated lysis.

In the preferred embodiment described herein, cells which have been genetically engineered can be transplanted into a host to allow them to both resist and evade the immune system of a host. The host will normally be a human or a domesticated farm animal.

Although described with reference to endothelial cells, especially dissociated endothelial cells for implantation or injection into a host, the methods and compositions described herein are not limited to endothelial cells. Other cell types can be similarly modified for transplantation. Examples of other cell types include fibroblasts, epithelial cells, skeletal, cardiac and smooth muscle cells, hepatocytes, pancreatic islet cells, bone marrow cells, astrocytes, Schwann cells, and other cell types, dissociated or used as tissue (i.e., organs). As described herein, "endothelial cells" will be construed to encompass modification of these other cell types unless otherwise specified or described specifically in the examples.

The cells can come from a variety of sources. Preferably, the cells are of non-human origin because of the ready availability of such cells in large quantities and at low cost. For example, the cells can be of porcine or bovine origin. Cells from primates, including humans, can be used if desired. Even if human cells are used, protection from hyperacute rejection will in general still be required since complement-mediated cell attack can also occur even following allotypic transplantation.

The genetically engineered cells are normally derived from a single clone or, for some applications, a group of individually selected clones. In this way, the characteristics of the final pharmaceutical preparation can be accurately controlled both in terms of the overall properties of the cells and their genetic make-up. Such control is of importance in evaluating the effectiveness of particular treatment protocols and in obtaining regulatory approval for such protocols.

The cells are genetically engineered so that they express a complement inhibitory protein or proteins on their cell surface. The cells are also genetically engineered so that they do not express on their cell surface proteins encoded by either the class I major histocompatibility complex genes or the class II major histocompatability complex genes which elicit a T-lymphocyte mediated reaction against the cell. Even when human cells are used, it is beneficial to engineer the cells as described herein since the cell population will generally include non-autologous cells when the cells are obtained from an individual other than the one being treated.

The endothelial cells are obtained from the lining of a portion of the vascular system, e.g., a blood vessel or capillary, and are grown and maintained in a tissue culture or other suitable biological medium.

For example, porcine large vessel endothelial cells are isolated from the thoracic aortae of male pigs.
1. The thoracic aortae is removed from the sacrificed animal using sterile techniques, cross-clamping the aortic arch and the aorta just above the renal arterial ostia using sterile clamps.
2. The organs/tissues are placed in sterile PBS buffer, containing 10X penicillin, streptomycin and fungizone. These are transported on ice.
3. After placing the aorta on a sterile field in a laminar flow clean bench, the peri-aortic fat and adventitial tissue are dissected away from the aortae. With an assistant holding the aorta down, using the clamps and a sterile scissors, the vessel is cut open longitudinally, exposing the endothelium. The endothelium is then rinsed with the sterile PBS/antibiotic-containing buffer.
4. Following this, the endothelium is scraped off with a sterile scalpel blade and the harvested endothelium is transferred into a sterile 15 cc conical centrifuge tube by displacing the cells with a stream of sterile PBS buffer. The tubes are centrifuged at 1200 RPM and the supernatant aspirated.
5. 5.0 ml of sterile media (DMEM, 10% heat-inactivated fetal calf sera, penicillin (100 U/ml), streptomycin (100 U/ml), 5 mM Hepes, 5 mM pyruvate and 5 mM glutamine, are added to each tube and the cell pellets resuspended in the media by gently pipetting the solution up and down in a sterile five ml pipette.
6. 5.0 ml aliquot of cells (harvested from each aorta) are placed into a Corning T25 tissue culture flask and the cultures incubated in a 5% CO₂, 95% air humidified atmosphere at 37°C.
7. The cells are then passaged at confluency at a 1 to 3 split ratio using 0.02% trypsin (Worthington Biochemical Corp.) in a Ca⁺⁺ and Mg⁺⁺-free PBS containing 0.01 % EDTA to dislodge the cells from the plate and dissociate the cells.

After being genetically engineered in the manner described below, the cells are normally stored in liquid nitrogen tanks until needed for the treatment of a particular patient. The ability to prepare the donor cells in advance and store them until needed is an important advantage.

Cells are then seeded onto a matrix for implantation. For example, dissociated endothelial cells are prepared for seeding onto the interior of Gortex™ as follows.
1. Sterile Gortex™ material is placed into a sterile Falcon™ 15 cc conical disposable test tube. Coating solution consisting of 0.1 M sodium carbonate buffer, pH 9.3, and 100 µg/ml acid soluble type I collagen is added to the test tube. Following an overnight incubation at 4°C, the Gortex™ is rinsed with sterile PBS. Type I collagen is used as a coating because it provides maximal, rapid endothelial cell adhesion (75% adhesion in 30 min and 80% adhesion in one hour) and migration. See, Madri, et al., "The collagenous components of subendothelium: Correlation of structure and function" Lab. Invest. 43:303-315 (1980).
2. The Gortex™ tubing is then cross-clamped at one end and endothelial cells axe introduced into the lumen of the Gortex™ tubing and the other end is cross-clamped.
3. The segment(s) of Gortex™ are then placed in a sterile tube and the tube filled with media and rotated at 5 rev/min in a 5% CO₂, 95% air humidified atmosphere at 37°C for one hour.
4. Remove the Gortex™ segments from the tubes and wash them with sterile media.
5. The Gortex™ segments are transplanted into the vasculature of the host.

### B. Treatment of Cells or Patients with a C5b-9 Inactivator to inhibit complement-mediated attack.

Alternatively, a C5b-9 inactivator can be administered in solution to cells or to a patient to inhibit complement-mediated attack. Administration or expression of the inhibitor, or a polypeptide representing its functional domain and possessing C5b-9 inhibitory activity produced from the isolated naturally produced inhibitor or from genetically engineered cells expressing (or more preferably, secreting) inhibitor, to block platelet or endothelial cell activation in a patient in need of such treatment, should thereby protect the patient from C5b-9 mediated procoagulant and prothrombotic responses.

Platelets obtained from patients with the acquired stem cell disorder Paroxysmal Nocturnal Hemoglobinuria (PNH) have been shown to exhibit abnormal sensitivity to fluid phase complement activation, as characterized by an unusually high risk of venous thrombosis. This same finding is equally applicable to other types of complement mediated disorders, particularly in view of the discovery that the inhibitor is also found on the surface of endothelial cells. As a result, administration of the inhibitor protein, whether purified from cells or expressed from cells engineered using recombinant techniques, or portions of the peptide having the same measurable activity, can be administered to these patients to alleviate the severity of the disorder.

Treatment of patients with immune disorders and diseases such as immunovasculitis, rheumatoid arthritis, scleroderma, disseminated inuavascular coagulation, lupus, paroxysmal nocturnal hemoglobins, thrombotic thrombolytic purpura, vascular occlusion, reocclusion after surgery, coronary thrombosis, and myocardial infarction, is accomplished by administering an effective amount of a composition containing a C5b-9 inactivator as defined above such that procoagulant processes are suppressed.

In the case of transfused blood cells, progenitor hematopoietic stem cells derived from or contained in bone marrow used for transplantation, or transplanted organs or tissue, the purified membrane inhibitor of C5b-9, or the functionally equivalent polypeptide or antibody, is first coated on the cell surface, or the gene introduced into the precursor cells as described above before transplantation or transfusion into the recipient. The precursor cells could be derived from the same species of origin as the recipient or from transgenic animals of a different species wherein the gene for CD59 for the recipient species is introduced into an embryo using techniques known to those skilled in the art such as microinjection. The amount of composition that must be administered to a patient in need of such treatment will vary depending on the particular disorder or disease and the severity of affliction. Treatment dosages will also vary with the individual patient depending upon response to treatment, genetic variability, and effect of co-administered drugs. In general, however, the compositions disclosed herein are administered intravenously at a dosage of approximately nanograms of inhibitory protein or peptide per milliliter, or gene expression used to effect surface expression of at least 1 x 10³ molecules/cell or 1 molecule CD59/µm². Treatment can take the form of a single administration of the composition or can be administered periodically or continuously over an extended period of time, as required. For treatment of immune disorder or disease, the C5b-9 inactivator is administered intravenously in a pharmaceutically acceptable carrier such as saline or a physiologically acceptable buffer. In some cases, it may be advantageous to administer CD59 in combination with genetically engineered cells to maximize effectiveness.

Isolated, functionally active polypeptides having the appropriate tertiary structure to inhibit C5b-9 have utility for increasing the hemostatic efficacy and extending the *in vitro* storage time of blood and platelet preparations. There exists a great need for prolonging the half-life, and therapeutic efficacy of platelets stored *in vitro*. Platelet-containing solutions, particularly platelet-rich plasma (PRP), are in tremendous demand medically for transfusions. The current shelf life of platelet preparations is approximately 72 hours. An increase in the useful lifetime of such preparations represents a significant advancement in the state of the art and answers a pressing human and medical need.

In the case of human organs and tissue for transplantation, the C5b-9 inactivators can be added to the pcrfusate or storage medium to protect the vascular lining cells from ongoing complement activation during *in vitro* storage. Additionally, by coating these endothelial cells with a membrane-anchored C5b-9 inactivator or inserting into the cells the gene for expressing the C5b-9 inactivator, as described in more detail below, the organ or tissue would be protected from the cytolytic and thrombotic effects arising from complement activation initiated upon transplantation, thereby circumventing complement mediated acute rejection.

In the preferred embodiment where the C5b-9 inactivator is administered alone, the C5b-9 inactivator is administered in combination with anticoagulant, such as ACD, CPD, heparin, or oxalate, such that the concentration in the platelets or PRP is approximately nanograms inactivator/ml, or expressed at a concentration of at least 1 x 10³ inhibitor/ml. Similarly, for organ storage, the C5b-9 inactivator is in combination with perfusate or storage solutions, or culture medium, such that the concentration is approximately nanograms inactivator/ml.

Compositions useful for extending the shelf life of platelet preparations stored *in vitro* contain C5b-9 inhibitor in an amount sufficient to inhibit C5b-9 mediated platelet activation. Generally, these compositions will be added to platelet preparations, such as platelet-rich plasma, such that the final concentration of inhibitory polypeptide in the preparation is in the range of greater than 2 Ki (Ki = concentration of half maximal inhibition) of the inactivator in the solution. For CD59 and other polypeptides which incorporate a membrane binding domain, the therapeutically effective dosage will be less than 1 µg inactivator/ml, or at least 1 x 10³ molecules inactivator/platelet or other cell. Useful compositions may also contain additional anticoagulant agents such as oxalate, citrate, and heparin. The C5b-9 inhibitor containing compositions can be added to whole blood as it is collected or to platelet preparations after processing of the blood into isolated platelet concentrates.

By increasing the surface concentration of these complement-inhibitors in the plasma membrane by increasing the level of transcript mRNA for the protein, the cells are protected from activated complement C5b-9 after infusion or tissue/organ transplantation.

### IV. Cell Termination

Since the engineered cells resist attack by the complement system and evade the T-cell system, the cells and their progeny in theory can exist essentially indefinitely within the host organism. Since occasions may arise when it is desirable to remove these cells from the host, further genetic engineering is preferably performed wherein the cells are provided with an in "self-destruct" or "suicide" mechanism.

In general terms, such a mechanism involves including in the cell a gene which expresses a protein, usually an enzyme, which confers lethal sensitivity of the cell to a specific reagent not normally present in the cell's environment. For example, the bacterial enzyme cytosine deaminase (CyD) converts the non-toxic drug 5-fluorocytosine to 5-fluorouracil which in turn is converted within the cell to 5-fluorouridine 5'-triphosphate and 5fluoro-2'-deoxyuridine 5'-monophosphate which inhibit both RNA and DNA synthesis and thereby result in cell death, as reported by Mullin, et al., 1992 "Transfer of the bacterial gene for cytosine deaminase to mammalian cells confers lethal sensitivity to 5--fluorocytosine: a negative selection system" Proc. Natl. Acad. Sci. USA 89:33-37.

Accordingly, by inserting the gene for bacterial CyD into the genome of the donor endothelial cell, cell death can be accomplished at any desired time by simply administering 5-fluorocytosine to the host organism. The sequence of the bacterial CyD gene is known and thus incorporation of the gene into the donor endothelial cells can be preformed in a manner similar to that used to insert the CD59 gene.

Other genes now known or subsequently identified, which confer lethal sensitivity to a selected material, can also be used for this purpose.

### V. Clinical Applications

As discussed above, the engineered cells can be used for cell replacement and for drug administration.

### Treatment of coronary artery disease.

For example, coronary artery disease is caused by a blockage inside blood vessels, reducing the delivery of oxygen and nutrients to the heart. The current treatment for coronary artery blockade is either to mechanically dilate the blocked vessel from the inside with an angioplasty balloon or to use a replacement vessel, e.g., a synthetic graft or a section of the saphenous vein, to bypass or form a new channel around the blockage.

Coronary angioplasty involves the insertion of a catheter from the leg vessel to the coronary artery and inflation of a balloon at the tip of the catheter to dilate the atherosclerotic plaque. This balloon inflation unfortunately has the undesired side effect of removing endothelial cells from the inner lining of the blood vessel.

In terms of clinical practice, reocclusion of the treated vessel following coronary angioplasty, i.e., restenosis, is a significant medical problem since it occurs within six months following 30-50% of the procedures performed and is associated with substantial patient morbidity and health care expenditures. The principal reasons for the restenosis are acute thrombus formation due to loss of the antithrombotic surface provided by the endothelial cells and neointima formation due to unchecked smooth muscle cell stimulation by blood-borne cells, again due to the loss of the protective endothelial cell layer.

Coronary bypass graft surgery does not involve removing the blockage to blood flow in the coronary artery, using instead a bypass to detour blood flow around the blocked vessel to supply the remainder of the heart muscle. When a portion of the saphenous vein is used to form the bypass, the inside lining of endothelial cells is normally stripped off the vessel wall, and the smooth muscle cells in the blood vessel wall injured.

The loss of the endothelial lining results in the loss of several critical endothelial properties including loss of the anticoagulant surface, loss of important smooth muscle cell regulatory force, and the loss of the protective vessel wall covering which shields smooth muscle cells from platelets, monocytes, and lymphocytes. The subsequent response of the blood vessel to this pathologic injury is two-fold: 1) the physiological and beneficial migration of endothelial cells from the edge of the wound to restore luminal integrity and 2) the pathophysiological migration of smooth muscle cells from the interior of the blood vessel wall toward the lumen resulting in the neointima formation and postintervention occlusion.

Occlusion of peripheral arterial and coronary artery bypass grafts is a frequent and important clinical finding. Two-thirds of the saphenous vein coronary bypass grafts are either severely diseased or entirely occluded by six to eleven years following bypass surgery. Peripheral arterial bypass grafts generally suffer occlusion within two to five years.

Synthetic grafts also exhibit high rates of occlusion. Initially, grafts of this type are not endothelialized. This results in a substantial incidence of early occlusion due to thrombosis. With time, the grafts become partially re-endothelialized by migration of arterial endothelial cells from the proximal and distal anastomotic sites or from ingrowth of capillary endothelial cells through the porous synthetic graft onto the luminal surface. However, the process of endothelial cell migration is normally slow and does not permit total coverage of the graft by arterial endothelial cells. Further, ingrowing capillary endothelial cells are less capable of inhibiting clot formation than arterial endothelial cells. Attempts to reseed peripheral grafts with autologous endothelial cells have demonstrated that incomplete coverage of the graft at the time of seeding results in graft closure and lack of clinical benefit of the seeding procedure.

The genetically engineered cells described herein provide an important mechanism for addressing these critical problems in revascularization. These cells can be used to re-endothelialize denuded vessels or grafts without significant rejection by the patient's immune system. Moreover, since the cells can be grown in large numbers before the surgical procedure, adequate supplies are available for coverage of large areas of denuded vessel or naked graft. In this connection, further genetic engineering of the endothelial cells can be performed in accordance with copending application Serial No. 07/820,011, filed January 6, 1992, entitled "Genetically Engineered Endothelial Cells Exhibiting Enhanced Migration and Plasminogen Activator Activity," the teachings of which are incorporated herein, so as to increase the rate of migration of the donor endothelial cells and thus achieve more rapid re-endothelialization.

A typical procedure for implanting universal donor endothelial cells in a patient's coronary artery is as follows:
1. Performing diagnostic catheterization of the patient to determine the severity, location and amenability of the coronary (or peripheral) artery disease to angioplasty, atherectomy, laser therapy, or other forms of mechanical revascularization.
2. Assuming step (1) determines that therapeutic angioplasty is appropriate, performing a standard balloon angioplasty procedure.
3. Removing the genetically engineered endothelial cells described herein from storage under liquid nitrogen, thawing the cells to 37°C and preparing them for installation by way of the angioplasty procedure by suspending them in sterile buffered media.
4. Using a standard wire exchange technique, removing the balloon angioplasty catheter and replacing it with a double balloon catheter having an infusion exit port positioned between the two balloons.
5. Positioning the double balloon catheter tip in the angioplastied coronary artery with the double balloons straddling the denuded segment of the artery, i.e., the portion of the artery in which the endothelial lining has been removed by the angioplasty procedure.
6. Gently inflating the double balloons while supporting the distal coronary circulation with standard perfusion techniques.
7. Introducing the engineered endothelial cells into the extracorporeal end of the double balloon catheter and infusing the cells into the isolated space in the blood vessel between the two balloons at a concentration of, for example, 2-10 x 10⁶ cells per 10 milliliters of solution, to seed the denuded portion of the vessel.
8. After approximately twenty to thirty minutes, deflating the double balloon catheter so as to restore normal antegrade coronary perfusion,
9. Removing the double balloon catheter followed by standard post catheterization procedures.

Similarly, a synthetic or autologous vascular graft or stent can be coated with genetically engineered endothelial cells and then implanted in a patient by:
1. Performing diagnostic catheterization of the patient to determine the severity, location and amenability of the coronary (or peripheral) artery disease to vascular bypass surgery with autologous, synthetic, or other graft material.
2. In the case of a synthetic graft or stent, such as a graft or stent made of DACRON or stainless steel, coating the graft or stent with Type I collagen and fibronectin in saturating amounts greater than or equal to 25 mg/ml in carbonate buffer, pH 9.4; or in the case of an autologous graft, harvesting the saphenous vein or other vessel using conventional surgical techniques.
3. Cannulating the proximal end and ligating the distal end of the synthetic or saphenous vein graft.
4. Removing the genetically engineered endothelial cells from storage under liquid nitrogen, thawing them to 37°C, and then preparing them for seeding of the graft by suspending them in sterile buffered media.
5. Injecting the engineered endothelial cells, at a concentration of, for example, 2-10 x 10⁶ cells per 10 milliliters of solution, through the proximal cannulation port into the lumen of the graft and rotating the graft for approximately 60 minutes to allow the universal donor endothelial cells to cover the graft surface.
6. Implanting the seeded graft in the coronary or peripheral artery using standard fine surgical techniques.

In addition to their use for cell replacement, the genetically engineered endothelial cells provide an excellent mechanism for the administration of therapeutic agents either locally at the site of cell implantation or systemically. These cells might also secrete PDGF or FGF antagonists, thrombolytics, or thrombin antagonists, so as to inhibit restenosis in a vessel or graft wall. Systemic drug delivery via universal donor endothelial cells might be most effectively accomplished by the use of genetically engineered microvascular (capillary) endothelial cells which offer several advantages including a relatively large surface area to volume ratio, especially when the cells are seeded into a capillary network as described below, and direct secretion of therapeutic protein products without any barrier to diffusion. Examples of the types of agents which can be administered in this way include blood clotting factors, clot dissolving factors, hormones, growth factors, cytokines, enzymes, and cholesterol binding or removing proteins. In each case, an appropriate gene or combination of genes is inserted into the genome of the donor endothelial cells prior to transplantation.

A typical procedure for isolating cells of this type, for example, from a porcine source, is as follows:
1. Porcine microvascular endothelial cells (PMEC) are isolated by first removing the epididymal fat pads and/or kidneys from male pigs using sterile techniques. To do this, the organs or tissues are placed in sterile HEPES buffer (pH 7.4) which contains 140 mM NaCl, 10 mM HEPES, 10 mm KCl, 0.1 mm CaCl₂, 0.2 mm MgCl₂, 11 g/liter NaHCO₃, 5.0 g/liter glucose, 100 U/ml penicillin, and 100 U/ml streptomycin. For kidneys, the peri-renal fat is dissected away and the kidneys are placed in sterile HEPES buffer as above.
2. The large visible vessels are dissected away from the epididymal fat and the fat is then placed into sterile HEPES buffer. The fatty tissue is placed into 50 ml sterile Falcon "Blue Max" tubes containing a small amount of sterile HEPES buffer and the fat is minced for 3 to 5 minutes with a scissors.
3. The minced tissue is then placed into 50 ml Erlenmeyer flasks containing equal volumes of sterile HEPES buffer containing 5 mg/ml of collagenase and 5 mg/ml of bovine serum albumin (BSA). The flasks are incubated at 37°C with agitation for 20 minutes. A small aliquot (0.1 ml) is removed from each flask every 20 minutes and then examined for the appearance of tube-like fragments of the capillary bed. The incubation is continued until the majority of the minced tissue contains tube-like fragments and single cells.
4. The cell suspension is centrifuged at 200 x g for 7 minutes in 15 ml sterile conical tubes. The top while fatty layer is then aspirated off and the pellets are resuspended in 10 ml of HEPES buffer containing 10% BSA and then recentrifuged and resuspended an additional two times.
5. The resultant pellets are resuspended in 45% Percoll and centrifuged at 15,000 x g for 20 minutes at 4°C in a SS34 fixed angle rotor. The tufts of the PMECs are in a milky off-white layer beneath the top-most adipocyte-containing layer and above a translucent layer containing larger vessel fragments. The microvascular tufts and free endothelial cells are collected with a sterile pipette and then pelleted by centrifugation in HEPES-BSA at 200 x g for 3 minutes. The tufts are resuspended in media (Medium 199E containing 20% heat-inactivated FBS, Penicillin, streptomycin, 5 mM HEPES, 5 mM Pyruvate, and 5 mm glutamine mixed 1:1 with the same medium containing 10% FBS which has been conditioned for 48 hours by incubating over confluent endothelial cell cultures).
6. The cells are then seeded into tissue culture flasks that have been coated with 1.5% gelatin in PBS overnight.
7. The PMEC cultures are then incubated in a 5% CO₂, 95% humidified atmosphere at 37°C. The PMEC are routinely passaged at confluency using 0.02% trypsin in a Ca²⁺ and Mg²⁺-free PBS containing EDTA to dislodge the cells from the plate and to dissociate cell aggregates.
8. Cells to be transfected/infected are plated at a 1 to 4 split ratio onto 75 ml Corning tissue culture flasks that have been coated with 1.5%. gelatin in phosphate-buffered saline overnight. After an overnight incubation, the cells are transfected/infected as described above for CD59.
9. The genetically engineered endothelial cells can be frozen under liquid nitrogen and stored until needed.
10. To seed the genetically engineered cells into a cell network, the engineered cells are first dispersed in a 5 mg/ml solution of neutralized acid soluble type I collagen (isolated from calf dermis) at a concentration of 3.0 x 10⁶ cells per ml of collagen solution at 4°C. This mixture is plated into 24-well cluster dishes in 0.75 ml aliquots and placed in a 37°C incubator with a 5% CO₂, 95% air humidified atmosphere. Following gelation of the collagen, media is introduced over the gels. The cells are then cultured as above, refeeding the cells on a daily basis. After three days, the gels are scraped off the dishes using a sterile Teflon™ cell scrapper and transferred into Bellco™ four liter suspension culture vessels for one week and then frozen under liquid nitrogen and stored until needed.

Because of their multi-level protection against the host's immune system, the engineered donor endothelial cells avoid graft rejection normally associated with the transplantation of non-autologous cells and thus can be used to administer their encoded therapeutic agent for substantial periods of time until, for example, removed from the host by a self-destruction mechanism of the type described above.

The present invention will be more fully described by the following non-limiting examples.

### Example 1: Methods for expression of human complement inhibiting proteins in mammalian cells, specifically human CD59 or functionally equivalent polypeptides.

Although described with specific reference to CD59, these methods are equally applicable to CD55 and CD46.

Materials: Human complement proteins C5b6, C7, C8 and C9 were purified and analyzed for functional activity according to methods described by Wiedmer and Sims, J. Biol.Chem, 260, 8014-8019 (1985). Human serum deficient in complement protein C8 (C*D) and the human complement proteins C8 and C9 were prepared and assayed according to Sims, P.J. Biochemistry 23,3248-3260 (1984), and Cheng, K., et al., J. Immunol. 135,459-464 (1985). Methotrexate was purchased from Lederle Laboratories (Carolina, Puerto Rico). BCECF/AM was from Molecular Probes (Eugene, OR). *N*-Glycanase was from Genzyme (Cambridge, MA). All other chemicals were of reagent or analytical grade.

### Solutions:

Hanks' balanced salt solution (HBSS) was purchased from Whittaker M.A. Bioproducts (Walkersville, MD) and made 1% (w/v) in fatty acid-free bovine serum albumin (Sigma).

### Antibodies:

Monoclonal antibody against CD59 (1F1) was obtained from Dr. Motowo Tomita (Showa University, Tokyo). Fab fragments of monospecific rabbit antibody against human erythrocyte CD59 were prepared as described by Sims, P.J., Rollins, S.A., and Wiedmer, T. (1989). Rabbit antiserum reactive with CHO membranes was prepared by repeated injection of plasma membranes derived from cultured CHO cells, and the IgG fraction (anti-CHO) was prepared by affinity purification using protein A-Sepharose (Sigma). Rabbit anti-human erythrocyte was purchased from Cappel (Cochranville, PA).

### Erythrocyte Membrane Protein Inhibitory for C5b-9 (CD59):

The 18 kDa human erythrocyte protein inhibitory for C5b-9 mediated activation and lysis, CD59, was isolated by modification of methods described by Sugita, et al. (1988). Additional purification was obtained by Mono-Q™ FPLC (Pharmacia). CD59 polypeptides having inhibitory activity can also be affinity purified using inhibitor specific antibodies. Antibodies, such as α-P18, which bind the C5b-9 inhibitor polypeptide, are immobilized on chromatographic matrix material by techniques well known to those skilled in the art, the material containing the 18 kDa protein passed over the chromatographic matrix, non-binding material removed by washing, then the bound material removed with a higher salt solution or similar technique. Polypeptides having the ability to inhibit C5b-9 mediated procoagulant responses are produced recombinantly. Nucleic acid sequences encoding CD59 or active fragments thereof, are isolated from a human cDNA library, or, preferably, the clone described herein. For example, human DNA is isolated and digested with restriction enzymes to create fragments of appropriate size and with appropriate cohesive ends to be ligated into any of the known and commercially available expression vectors (e.g. Promega's lambda gt11 vector system). Alternatively, the isolated DNA is sheared and the appropriate linkers are ligated onto the resulting fragments which are then inserted into the expression vector of choice.

Vectors containing human DNA fragments are next transformed into the appropriate bacterial strain, normally a strain of *E. coli* that is included in the expression vector kit, to generate the DNA gene bank or library. Plating out the vector containing bacteria of the library on appropriate media results in expression of the inserted human DNA fragment. The colonies are screened for the presence of DNA encoding and expressing the C5b-9 inhibitory polypeptide using specific antibodies such as α-P18. Positive colonies are isolated and used for the large scale expression of recombinanuy produced inhibitory protein.

In this fashion intact inhibitory protein can be made recombinantly as well as modified polypeptides and functional fragments and derivatives thereof. Functional polypeptides possessing the tertiary structure and ability to inhibit C5b-9 can be produced by any of the above discussed method or by other techniques commonly known to those of ordinary skill in the art. These isolated and purified polypeptides can be further mixed with pharmaceutically acceptable carriers to form compositions for use in prolonging cell storage or in treatment of immune disorders or diseases.

The following methods are useful in detecting and quantitating C5b-9 inhibitory activity of CD59 or fragments thereof.

### Protein Labeling for fluorescence or radiolabelling:

For flow cytometry, all antibodies were conjugated with fluorescein isothiocyanate (FITC). The IgG fraction of affinity-purified goat antibody against murine IgG (Sigma) was labeled with FITC anti-mouse IgG. Dye:protein ratios range from 3 to 6. In all cases, unincorporated label (¹²⁶I or FITC) is removed by gel filtration followed by exhaustive dialysis.

Monoclonal antibody 1F1 was radiolabeled with IODO-GEN™ (Pierce Chemical Co.) to a specific activity of 6221 cpm/ng.

### Protein Concentrations:

Concentrations of unlabeled proteins are estimated assuming the following extinction coefficients (E^{1%}₂₈₀): murine IgG (15), C8 (15.1), and C9 (9.6). The concentrations of FITC-labeled proteins are determined by dye binding assay (BioRad), using the respective unlabeled protein as standard. FITC concentration is determined assuming a molar extinction (492 nm) of 68,000.

### Western Blotting

Purified human erythrocyte CD59 (1 µg) and the antigen from CD59 transfected CHO cells were denatured (3 min, 100°C) in 2% SDS under nonreducing conditions and electrophoresed in a 15% homogenous gel using a Laemmli, U.K. Nature 227, 680-685 (1970), buffer system. Following transfer to nitrocellulose, immunoblotting was performed by overnight incubation at 23°C with either monoclonal anti-CD59 (10 µg/ml) or rabbit anti-CD59 (10 µg/ml) in TBS (150 mM NaCl, 50 mM Tris, pH 7.4) with 1% bovine serum albumin. Blots were developed with a 1:1000 dilution of the appropriate alkaline phosphatase-conjugated anti-rabbit or anti-mouse IgG (Sigma).

### Transfection and Expression of CD59 cDNA in CHO Cells.

The *EcoRI* fragment that encodes the CD59 protein, described by Philbrick, W.M., et al., Eur. J. Immunol. 20, 87-92 (1990, was subcloned into the *EcoRI* site in the µFRSV expression vector, reported by Slanetz, A.E., and Bothwell, A.L.M. Eur. J. Immunol. 21, 179-183 (1991).

The amino acid sequence for the protein encoded by this insert is:

The cDNA sequence encoding the CD59 protein is:

The FRSV.CD59 vector was linearized with *Sal*I (20 µg) and introduced into 10⁷ CHO cells by electroporation (2 kV, 25 microfarads). The cells were plated in minimum Eagle's medium (GIBCO) containing 10 mg/ml adenosine, thymidme, and deoxyadenosine and maintained for 1 to 2 days. The medium was then replaced with minimum Eagle's medium lacking deoxynucleosides but containing 0.09 µg/ml methotrexate and 10% dialyzed fetal calf serum (GIBCO). After 2 to 3 weeks, individual clones were isolated, expanded, and selected at increasing levels of methotrexate.

The resulting transfectants were subcloned and selected for growth in medium that is supplemented with methotrexate and then amplified by continuous culture in incremental concentrations of methotrexate ranging up to 1 mg/ml, as shown in Figure 1.

Immunoblotting of CD59 expressed by transfected CHO cells and human erythrocytes was performed. Immunoblots were developed with monoclonal antibody 1F1 (10 µg/ml) and rabbit anti-CD59 IgG (10 µg/ml). The transfected CHO cell-derived protein had a greater molecular weight than the native molecule.

Based on the specific binding of radiolabeled monoclonal antibody against this antigen, cell-surface CD59 was increased from 0 (in nontransfected CHO controls) to approximately 4.2 x 10⁶ molecules/cell (for those CD59 transfectants maintained in 1 mg/ml methotrexate).

### Quantitation of Cell-surface CD59 Antigen

The specific binding of ¹²⁵I-labeled 1F1 was utilized to quantitate the level of CD59 antigen expressed by CD59-transfected CHO cells. The CHO cells (CD59 transfectants and controls) were grown to confluence in 48-wall tissue culture plates, washed in HBSS, and then fixed with 1% paraformaldehyde (10 min, 23°C). After washing to remove fixative, the cells were incubated with a saturating concentration of antibodies, 10 µg/ml ¹²⁵I-1F1, for 30 min at 23°C. The cells were then washed six times in ice-cold HBSS, and cell-associated antibody was eluted with 4% sodium dodecylsulfate (SDS). Radioactivity was measured by photon counting and corrected for nonspecific binding, measured in the presence of a 20-fold excess of unlabeled antibody. Data for CD59 transfectants was expressed as increase in surface antigen relative to nontransfected CHO cell controls.

Subcloning of a cDNA clone for human CD59 into the eukaryotic expression vector pFRSV and transfecting into CHO cells via electroporation demonstrates that CD59 can be expressed in cells not normally expressing CD59 or HRF and confer protection from complement mediated lysis. Although pFRSV was chosen to incrementally amplify the DNA flanking the dihydrofolate reductase locus in a methotrexate concentration-dependent manner, other vectors could also be used such as pFRSV-SRα.

The vector pFRSV has been used successfully to increase expression after gene amplification by selection in methotrexate containing medium. The plasmid vector pFRSV-SRα has a much stronger promoter driving expression of the introduced cDNA. The SalI-EcoRI fragment (800 bp) from pcDL-SRα296 (Takebe, et al., Molec. Cell Biol. 8:466-472 (1988)) was inserted into the *Hind*III-*Eco*RI site of pFRSV, described by Slanetz and Bothwell, (1991). This plasmid expresses much higher basal levels of CD59 and other inserted cDNAs than the pFRSV vector.

Expression in mammalian cells, especially endothelial cells, may also be accomplished by infection utilizing retrovirus vectors. This method is more gentle and efficient than electroporation as a means of introducing the DNA into cells. Retroviruses that bear different drug resistance markers for selection provides a means for introducing multiple cDNAs for expression in endothelial cells. Retroviruses currently under development for this purpose include the use of the neo, hygrogycin and histidinol as selectable markers. All of these resistance genes are available on *Bam*HI fragments and can be easily inserted into retroviral vectors to alter the resistance of a given vector. The DHFR gene is also available in retrovirus vectors. Retroviruses that express CD59 driven by the SRα promoter or the retroviral LTR (long terminal repeat) promoter can be utilized.

The use of retroviruses that bear distinct drug resistance markers can facilitate the co-expression of CD59 with either DAF(CD55) or MCP(CD46). Co-expression with CD59 may be more effective than CD59 alone in minimizing endothelial cell activation. Although desirable, it is not essential for the retrovirus vector to bear a drug resistance marker. Retroviruses may be developed that express both CD59 and CD55 or CD46 from a single virus. It is also possible to utilize vectors bearing different drug markers for expression of all three complement regulatory proteins CD59, CD55 and CD46.

### Molecular weight comparison of recombinant, natural, and de-glycosylated CD59.

Membrane proteins from CD59-transfected CHO cells (CD59 expression amplified by growth in 1 mg/ml methotrexate) were extracted with 2% Triton X100, 20 mM Tris, 10 mM EDTA, 50 mM benzamidine, 200 mM *N*-ethylmaleimide, 1 mM phenylmethylsulfonyl fluoride, pH 7.4. After removal of insoluble material by centrifugation at 11,000 x g, 5 min, the detergent extracts were diluted 10-fold, and CD59 antigen was purified by immunoaffinity chromatography using antibody immobilized on Affi-Gel 10 (Bio-Rad). Antigen was eluted with 1 M glycine, 0.2% Triton X-100, 10 mM EDTA, 50 mM benzamidine, 200 mM *N*-ethylmaleimide, 1 mM phenylmethylsulfonyl fluoride, pH 3.0; dialyzed against 200 mM sodium phosphate, 10 mM EDTA, 50 mM benzamidine, 200 mM *N*-ethylmaleimide, 1 mM phenylmethylsulfonyl fluoride, pH 8.6; and concentrated to 200 µg/ml. After denaturation under reducing conditions (0.5% SDS, 100 mM β-mercapoethanol; 100°C, 3 min), immunoaffinity-purified CD59 was incubated (37°C, 24 h) with 30 units/ml of *N*-glycanase in the presence of 10 mM 1,10-phenanthroline and then analyzed by silver staining after 8-25% SDS-PAGE (PHAST System, Pharmacia LKB Biotechnology Inc.).

Recombinant CD59 expressed on the surface of these cells was susceptible to removal by phospharidylinositol-specific phospholipase C digestion, consistent with its attachment to the membrane via a glycolipid anchor, as shown in Figure 2. Confluent monolayers of CD59-transfected CHO cells maintained in 1.0 mg/ml methotrexate were released from T-25 tissue culture flasks with Versene/EDTA (Whittaker M.A. Bioproducts). After washing and suspension to 2 x 10⁶ cells/ml in HBSS, these cells were exposed to 1.0 unit/ml phosphatidylinositol-specific phospholipase C (ICN Biochemicals, Indianapolis, IN) or enzyme diluent buffer as a control. After incubation for 1 h at 37°C, 25 µl of each cell suspension was added to tubes containing 25 µl of monoclonal antibody 1F1 (final concentration 10 µg 1F1/ml in HBSS). After 30 min of incubation at 4°C, 10 µl of FITC anti-mouse IgG was added (final concentration, 87 µg IgG/ml), and cells were incubated for an additional 15 min at 23°C. Surface CD59 was quantitated by specific binding of monoclonal antibody 1F1, measured by a FACSCAN (Becton, Dickinson & Co.) flow cytometer with the FL1 fluorescence channel (520 nm) set at logarithmic gain.

By Western blotting, CD59 expressed by the transfected CHO cells exhibited a distinctly slower migration on SDS-PAGE (apparent molecular mass of 21-24 kDa) than CD59 present in human erythrocytes (apparent molecular mass of 18-21 kDa). After digestion with *N*-glycanase to remove asparagine-linked carbohydrate, recombinant CD59 isolated from CHO transfectants and CD59 isolated from human erythrocytes co-migrated, with apparent molecular masses of 12-14 kDa by SDS-PAGE.

### Protection of CD59-transfected CHO Cells from Pore-forming Activity of Human C5b-9.

The functional activity of recombinant CD59 expressed in the transfected CHO cells was evaluated by assaying complement-mediated dye release using the intracelhilar fluorescent dye indicator BCECF/AM. By taking advantage of the capacity to incrementally amplify gene expression by growth in various concentrations of methotrexate, as shown in Figure 1, the inter-relationship of the CD59 antigen level to C5b-9 inhibitory activity was evaluated.

After stable expression at each methotrexate concentration was achieved, the CD59 transfected cells were tested for sensitivity of human serum complement using a BCECF/AM dye release assay. After incubation with BCECF/AM (15 µM) and washing, confluent monolayers were incubated with 5 mg/ml rabbit anti-CHO IgG and 25% C8D to deposit C5b-67 on the plasma membrane. Then, human serum containing 10 mM EDTA was added as the source of C8 and C9. Dye release into the supernatant was determined after 15 min at 37°C, with correction for nonspecific release observed for matched controls, omitting incubation in C8D.

As shown in Figure 3, increased amplification of the expression of CD59 resulted in a marked decrease in the sensitivity of the transfected CHO cells to dye release induced by immune activation of human serum complement. For cells grown in 200 µg/ml methotrexate (representing amplification of cell-surface expression to approximately 1.2 x 10⁶ molecules of CD59/cell), no complement-mediated dye release was observed, even at the highest concentrations of serum tested (75%).

To demonstrate complement inhibitory activity, CD59 expression of transfected CHO cells was amplified by growth in 50 µg/ml methotrexate: the cells were loaded with dye by incubation in BCECF/AM; and C5b-67 was deposited as described for Figure 3. After washing, the cells were incubated (4°C, 30 min) with either 0 mg/ml or 0.5 mg/ml functionally inhibitory antibody (Fab fragments) to CD59. Unbound antibody was removed; C8 (1 µg/ml) and varying amounts of C9) were added; and dye release was measured after 15 min at 37°C.

The resistance to complement-mediated membrane damage observed for CD59-expressing CHO cells reflected inhibition of C9-dependent activation of the complement pore, and this inhibition was reversed by prior incubation of the cells with Fab fragments of a functionally blocking antibody directed against CD59 antigen. These data confirm that the protection against human serum complement observed for CD59 transfectants is related to the expression of cell-surface CD59 and is not due to other changes in these cells that might be induced by long-term culture in methotrexate.

### Human Selectivity of Complement Inhibitory Function Expressed by CD59-transfected CHO Cells.

CD59-transfected CHO cells are selectively protected from the effects of the human C5b-9 proteins in a manner analogous to the species-selective resistance to lysis observed for human erythrocytes and for membranes reconstituted with purified CD59 antigen from human erythrocytes. In these studies, anti-CHO IgG and human C8D were used to deposit the human C5b-67 complex on the CHO cell plasma membrane, before incubation in either human or guinea pig serum containing EDTA, as the source of the C8 and C9 components of the C5b-9 complex.

CD59 expression by transfected CHO cells was amplified by growth at various methotrexate concentrations; the confluent monolayers were loaded with BCECF/AM; and human C5b-67 was deposited as described above. After washing, the C5b67 cells were made C5b-9 by incubation (15 min, 37°C) with either 10% (v/v) human serum (closed symbols) or 10% v/v) guinea pig serum (open symbols) in the presence of 10 mM EDTA.

CD59 expressed by transfected CHO cells protected these cells from pore formation by human C5b-9, but not when the C8 and C9 components of this complex were replaced by the guinea pig proteins.

Human C5b-67 was deposited on K562 cells by successive incubation with anti-human erythrocyte antiserum (1.5% (v/v, containing 10 mM EDTA) and 60% (v/v) human C8-depleted serum (diluted in HBSS). After loading with BCECF/AM cells were incubated (4°C, 30 min) with either 0 mg/ml or 1 mg/ml of functionally- blocking antibody to CD59. After removal of unbound antibody, the cells were made C5b-9 by incubation in either human (circles) or guinea pig (triangles) serum containing 10 mM EDTA. This capacity to restrict the pore-forming activity arising upon incorporation of human (but not guinea pig) C8 and C9 into C5b-9 was also observed for CD59 constitutively expressed on the surface of human K562 cells, the cell line from which the cDNA for CD59 used in these studies was originally derived.

The effective potency of CD59 and other inhibitors of the C5b-9 complex depends on the number of C5b-9 complexes generated per unit area of plasma membrane. Therefore, the inhibitory effect of CD59 on the cytolytic and cell-stimulatory activity of C5b-9 can be overcome by increasing the input of the activated complement components that are required for assembly of the C5b-9 complex. The protective effects of CD59 can be augmented by providing CD59 in conjunction with CD46 and/or CD55. In this formulation, CD59 is expressed in conjunction with CD46 and/or CD55 by transfection or infection with a vector containing the gene for each protein. The co-expression of these genes will serve to limit the amount of C5b-9 that can be generated at the cell surface (through the inhibitory effects of CD46 and/or CD55 on the conversion of C5 to C5b by the complement enzymes) and to protect from the cytolytic and cell-stimulatory effects of the residual C5b-9 that is formed through conversion of C5 to C5b by plasmin and other enzymes that are not inhibited by the action of CD46 and/or CD55.

### Example 2: Expression of Human CD59 in Porcine Endothelial Cells Protects them from Hyperacute Rejection by Human Complement.

This example demonstrates that when the full-length cDNA encoding the human CD59 protein is stably incorporated into the genome of a porcine aortic endothelial cell (PAEC) and expressed on the cell surface, it protects these cells from complement-mediated attack as assayed by human complement-mediated cell lysis *in vitro.*

Cultures of PAEC were cultured in DMEM containing 10% fetal bovine serum (FBS), 5 mM Hepes, 2 mM L-glutamine, and 1% each of penicillin and streptomycin (P/S). Prior to retroviral infection, the cells were grown to 50% confluence. Subconfluent PAEC were infected by using the amphotropic helper-free retroviral vector pRNSRalphaCD59+. The structure of this retroviral construct is shown in Figure 4. As controls, PAEC were also infected with a control retroviral vector containing the drug selection marker gene neomycin or were uninfected. The amphotropic retroviral stocks were added to subconfluent cells growing in a T-25 tissue culture flask in a total volume of 3 ml. Polybrene was added to the flasks and the cultures were incubated at 37°C for 2 to 5 hours. The cell culture media was then removed, monolayers were rinsed two times in 5 ml of media and then 5 ml of media was added to the cells which were incubated at 37°C in 8% C02.

After a 24 to 48 hour incubation period, the cells were exposed to G418 (400 µg/ml) to select for stable integration of the retroviral construct. Neomycin resistant colonies were assayed for the cell surface expression of human CD59 by flow cytometric techniques (FACS analysis). To assess cell surface expression of human CD59 on porcine endothelial cells, confluent neomycin-resistant cell clones were grown to confluence in T-75 tissue culture flasks and cells were released for FACS analysis by incubation in Versene-EDTA for 10 minutes at 37°C. Harvested cells were pelleted via centrifugation and then resuspended in 2 ml of staining buffer containing phosphate-buffered saline (PBS), 0.2% sodium azide, and 2% FBS. Cells were then counted with a hemacytometer, pelleted by centrifugation, rinsed two times with staining buffer and then incubated for 30 minutes at 23°C with a primary antibody to human CD59, either polyclonal rabbit anti-CD59 at 10 µg/mi or mouse anti-CD59 monoclonal 1F1 (obtained from Dr. Motowa Tomita, Showa University, Japan) at 1 µg/ml. The cells were then rinsed two times in staining buffer and then incubated for 30 minutes at 23°C with an FTTC-conjugated goat anti-rabbit IgG or an anti-mouse IgG diluted 1:50 in staining buffer. The cells were rinsed two times in staining buffer, once in PBS and then resuspended in 1% paraformaldehyde in PBS and analyzed by FACS. Positive cell surface expression of human CD59 (as measured by fluorescence intensity on the x axis) is demonstrated in Figure 5 for cell clones 1, 2 and 9 but not for control PAEC infected with control containing only the neomycin resistance gene.

With regard to their biological behavior, the CD59-infected PAEC were not different from either uninfected PAEC or PAEC infected with control vector. For example, they maintained proliferation rates identical to uninfected cells and they did not overgrow monolayers or proliferate in suspension, and were contact inhibited. Additionally, CD59-infected porcine endothelial cells were capable of attaching to a synthetic Gortex™ graft, as demonstrated in the scanning micrograph shown in Figure 6. Two centimeters square of synthetic Gortex™ sheets were steam-sterilized, placed in sterile 35 mm bacteriological petri dishes and overlaid with sterile stainless steel fences having a one centimeter square well. CD59-infected PAEC were then seeded into the center wells of the fences at a density of 1 x 10⁵ cells in a volume of 0.5 ml of culture media as described above and incubated at 37°C in 5% CO₂. After two days, the cultures were refed with media and after an additional two days the media was aspirated off and the cultures were washed with PBS and then fixed with buffered 2% glutaraldehyde, 4% paraformaldehyde for one hour. The fences were then removed and the Gortex™ was processed for scanning electron microscopy. Figure 6 demonstrates that PAEC expressing cell surface human CD59 attach as well to synthetic Gortex™ grafts as normal endothelial cells.

With regard to their biological activity, CD59-infected PAEC were assayed for their sensitivity to cytolysis by complement in human serum. To do this, CD59-infected PAEC, control PAEC infected with vector alone, and uninfected PAEC were plated into 48-well tissue culture plates at a density of 1.25 x 10⁵ cells/well in DMEM with 10% FBS, 2 mM glutamine and P/S. The culture media was removed and the cells were washed three times with media without FBS. Next, human serum diluted in DMEM at various concentrations was added to the cultures for 2 hours at 37°C. The percentage of viable cells remaining in the cultures was assessed by staining the cells with 0.1% trypan blue. Figure 7 demonstrates that greater than 80% of uninfected or control (vector alone infected) PAEC were killed by human serum whereas less than 10% of CD59-infected PAEC were killed. These results demonstrate that human CD59 expression on the surface of porcine endothelial cells protects these cells from the cytolytic activity of antibody and human serum, suggesting that *in vivo* these cells would be protected from complement-mediated hyperacute rejection.

### Example 3: Disruption of the Invariant Chain Gene by Homologous Recombination Gene Targeting

This example demonstrates that the invariant chain gene can be disrupted in mouse embryonic stem cells by specifically and stably replacing it in the genome with a mutated and non-functional form of the invariant chain gene and that replacement of the native invariant chain gene with a non-functional mutant can be achieved in a given cell by gene targeting technology which takes advantage of a homologous recombination event between the mutated gene and the native invariant chain gene. A partial restriction enzyme map for the mouse invariant chain gene is shown in Figure 8B. Digestion of mouse genomic DNA with the restriction enzyme *Dra*III should generate an invariant chain gene fragment of approximately 8.7 kb when this DNA is probed by Southern blotting with a radiolabeled probe specific for the mouse invariant chain gene. This result is obtained and demonstrated in Figure 9 (lane identified as parental cells).

To disrupt the mouse invariant chain gene by homologous recombination, a gene targeting vector was constructed, so as to replace a sequence of the invariant chain gene between nucleotides 661 and 1064 with the neomycin gene. This genetic engineering leads to the elimination of most of exon 1 including the translation initiation codon ATG, and a large portion of the promoter including the TATA box and CART box which function as regulatory elements required for accurate and efficient transcription of the invariant chain gene, as reported by Zhu and Jones, 1989 "Complete sequence of the murine invariant chain (Ii) gene" Nucleic Acids Res. 17:447-448. This gene targeting vector is shown in Figure 8A as a general example of the disruption strategy. By deleting this region of the invariant chain gene, all expression of this gene including transcription and translation will be eliminated. It follows, therefore, that the expression of class II MHC gene products will be disrupted.

Replacement of the native invariant chain gene with the mutated invariant chain gene was demonstrated in mouse embryonic stem cells. Embryonic stem cells (ES cells) were routinely passaged every other day in ES growth media containing DMEM (high glucose) with 15% FBS and 0.1 mM 2-mercaptoethanol. The ES cells were maintained on a confluent layer of primary embryonic fibroblasts. Two days prior to the transfection of the ES cells with the gene targeting vector the cells were expanded in culture. To transfect these cells, 25 µg of DNA corresponding to the invariant chain targeting vector were introduced into 1 x 10⁷ ES cells by electroporation using a BioRad electroporator set at 250 µF and 0.32 kV. The ES cells were then seeded onto 10 x 100 mm Nunc™ tissue culture plates and stable transfectants were selected for chromosomal integration by way of neomycin resistance in G418 (170 µg/ml) and/or gangcyclovir in some experiments where the herpes-simplex virus thymidine kinase gene was included in the targeting vector.

After 14 days in selection media, individual neomycin resistant colonies were selected and propagated on feeder layers of confluent embryonic fibroblasts. Genomic DNA was isolated from 55 individual stable transfectants and digested overnight with either the *Eco*RI or *Dra*III restriction endonucleases. Digested DNA was resolved by electrophoresis, blotted to GeneScreen+™ nylon membranes and then hybridized with a radiolabeled DNA probe specific for the mouse invariant chain gene. The pattern of hybridizing bands for two independent clones, 11.10.93 and 11.10.128, shown in Figure 9 is consistent with the mutant form of the invariant chain gene (where exon 1 was disrupted by the insertion of neomycin gene sequence) having homologously recombined and replaced the endogenous invariant chain gene. The pattern of restriction nucleate digestion observed is outlined diagrammatically in Figure 8C. The frequency of recombination was determined to be 2 in 55 clones. These data, therefore, demonstrate that this targeting vector can disrupt the native cellular invariant chain gene.

## Claims

1. A genetically engineered mammalian cell for transplantation into a human or animal
wherein the cell does not express on its surface proteins encoded by either the class I major histocompatibility complex genes or the class II major histocompatability complex genes which elicit a T lymphocyte mediated reaction against the cell and
the cell expresses a stably incorporated nucleotide molecule which codes for a protein inhibiting complement mediated attack of the engineered cell when introduced into an animal of another species or another individual.

2. The cell of claim 1 wherein the nucleotide molecule codes for a protein inhibiting complement mediated attack in the same species as the species the cell is derived from and the nucleotide molecule is located at a separate loci than the naturally occurring gene encoding the same molecule within the cell.

3. The cell of claim 1 wherein the promoter of the gene encoding a protein inhibitor of complement mediated attack on the host cell containing the gene is altered to increase expression of the gene within the host cell.

4. The cell of claim 1 wherein the protein inhibiting complement mediated attack is selected from the group consisting of CD59, CD46 and CD55.

5. The cell of claim 1 selected from the group consisting of endothelial cells, fibroblasts, epithelial cells, skeletal, cardiac and smooth muscle cells, hepatocytes, pancreatic islet cells, bone marrow cells, astrocytes and Schwann cells.

6. The cell of claim 1 wherein the cell is of non-human origin, the protein inhibiting complement is a human protein and the MHC class II genes are selected from the group consisting of the cell species equivalent of HLA DP, DR and DQ.

7. The cell of claim 1 wherein the cell is of non-human origin, the protein inhibiting complement mediated attack is a human protein and the MHC class I genes are selected from the group consisting of the cell species equivalent of HLA A, B and C.

8. The cell of claim 1 wherein the cell does not express the class II MHC proteins as a result of disruption of the invariant chain gene of the class II MHC genes.

9. The cell of claim 1 wherein the cell does not express the class I MHC proteins as a result of disruption of the β2-microglobulin gene.

10. The cell of claim 1 further comprising a nucleotide molecule which is expressed by the cell and which codes for a protein which in the presence of a selected compound results in cell death.

11. The cell of claim 10 wherein the nucleotide molecule codes for bacterial cytosine deaminase.

12. The cell of claim 1 wherein the cell is selected from the group consisting of cells of human, bovine and porcine origin.

13. The cell of any of claims 1-4 or 6-12 selected from the group consisting of hematopoietic progenitor cells, hematopoietic progenitor cell progeny and the mature blood cells.

14. The cell of any of claims 1-13 wherein the cell expresses a nucleotide molecule which codes for a therapeutic agent, which is secreted by the cell and the cell does not normally express the molecule, express the molecule from the same promoter or expresses the molecule from multiple loci.

15. The cell of any of claims 1-14 expressing on its surface greater than or equal to 10³ CD59 molecules/cell or greater than or equal to 1 molecule of CD59 antigen/µm² of plasma membrane surface.

16. The cell of claim 14 wherein the cell is a microvascular endothelial cell.

17. The cell of claim 1 which is a mouse cell.

18. The cell of claim 17 wherein the cell does not express the class II MHC proteins as a result of disruption of the invariant chain gene of the class II MHC genes.

19. The cell of claim 17 wherein the cell does not express the class I MHC proteins as a result of disruption of the β2-microglobulin gene.

20. A transgenic mouse containing any of the cells of claims 17.to 19.

21. An organ from a transgenic mouse, the organ formed of cells of any of claims 17 to 19.

22. A prosthesis for implantation in an animal or human having cells of any of claims 1-16 attached thereto.

23. The prosthesis of claim 22 wherein the prosthesis is a vascular graft and the cells are microvascular endothelial cells.

24. A method for re-endothelializing a denuded blood vessel *ex vivo* comprising applying to the vessel cells of claim 16 or the prosthesis of claim 23.

25. A cell according to any one of claims 1 to 16 or an organ according to claim 21 or a prosthesis according to claims 22 or 23 for use in medicine.

26. Use of the cells of claim 16 or the prosthesis of claim 22 in the manufacture of an agent for re-endothelializing a denuded blood vessel.

## Patentansprüche

1. Genetisch veränderte Säugerzelle zur Transplantation in einen Menschen oder ein Tier,
worin die Zelle auf ihrer Oberfläche keine Proteine exprimiert, die entweder durch Klasse I Haupthistokompatibilitätskomplexgene oder Klasse II Haupthistokompatibilitätskomplexgene codiert werden, welche eine T-Lymphozyten-vermittelte Reaktion gegen die Zelle auslösen, und
worin die Zelle ein stabil eingebautes Nukleotidmolekül exprimiert, das für ein Protein codiert, das einen Komplement-vermittelten Angriff der veränderten Zelle inhibiert, wenn sie in ein Lebewesen einer anderen Spezies oder ein anderes Individuum eingebracht wird.

2. Zelle nach Anspruch 1, worin das Nukleotidmolekül für ein Protein codiert, das einen Komplement-vermittelten Angriff in der gleichen Spezies wie der Spezies, von welcher die Zelle stammt, inhibiert und worin das Nukleotidmolekül an einem unterschiedlichen Locus lokalisiert ist wie das natürlich auftretende Gen, das das gleiche Molekül innerhalb der Zelle codiert.

3. Zelle nach Anspruch 1, worin der Promotor des Gens, das für einen Proteininhibitor eines Komplement-vermittelten Angriffs auf die das Gen enthaltende Wirtszelle codiert, verändert ist, um die Expression des Gens innerhalb der Wirtszelle zu erhöhen.

4. Zelle nach Anspruch 1, worin das Protein, das einen Komplement-vermittelten Angriff inhibiert, ausgewählt ist aus der Gruppe, bestehend aus CD59, CD46 und CD55.

5. Zelle nach Anspruch 1, ausgewählt aus der Gruppe, bestehend aus Endothelialzellen, Fibroblasten, Epithelzellen, Skelett-, Herz- und glatten Muskelzellen, Häpatocyten, Pankreasinselzellen, Knochenmarkszellen, Astrocyten und Schwanh'schen-Zellen.

6. Zelle nach Anspruch 1, worin die Zelle nicht menschlichen Ursprungs ist, das Komplement-inhibierende Protein ein menschliches Protein ist und die MHC-Klasse II-Gene ausgewählt sind aus der Gruppe, bestehend aus der Zellspezies, die äquivalent HLA DP, DR und DQ ist.

7. Zelle nach Anspruch 1, worin die Zelle nicht menschlichen Ursprungs ist, das Protein, das Komplement-vermittelten Angriff inhibiert, ein menschliches Protein ist und die MHC-Klasse I-Gene ausgewählt sind aus der Gruppe, bestehend aus der Zellspezies, die äquivalent HLA A, B und C ist.

8. Zelle nach Anspruch 1, worin die Zelle die Klasse-II-MHC-Proteine als ein Ergebnis einer Disruption des invarianten Kettengens der Klasse II MHC-Gene nicht exprimiert.

9. Zelle nach Anspruch 1, worin die Zelle die Klasse-l-MHC-Proteine als ein Ergebnis einer Disruption des β2-Mikroglobulingens nicht exprimiert.

10. Zelle nach Anspruch 1, weiterhin umfassend ein Nukleotidmolekül, welches durch die Zelle exprimiert wird und welches für ein Protein codiert, welches in der Gegenwart einer ausgewählten Verbindung zum Zelltod führt.

11. Zelle nach Anspruch 10, worin das Nukleotidmolekül für bakterielle Cytosindeaminase codiert.

12. Zelle nach Anspruch 1, worin die Zelle ausgewählt ist aus der Gruppe, bestehend aus Zellen, die ihren Ursprung bei Mensch, Rind und Schwein haben.

13. Zelle nach einem der Ansprüche 1 bis 4 oder 6 bis 12, ausgewählt aus der Gruppe, bestehend aus hämatopoetischen Vorläuferzellen, Nachkommen von hämatopoetischen Vorläuferzellen und den reifen Blutzellen.

14. Zelle nach einem der Ansprüche 1 bis 13, worin die Zelle ein Nukleotidmolekül exprimiert, welches für ein therapeutisches Mittel codiert, welches durch die Zelle sekretiert wird und worin die Zelle das Molekül normalerweise nicht exprimiert, das Molekül vom gleichen Promotor exprimiert oder das Molekül aus mehreren Loci exprimiert.

15. Zelle nach einem der Ansprüche 1 bis 14, die auf ihrer Oberfläche mehr oder gleich 10³ CD59-Moleküle/Zelle oder mehr oder gleich 1 Molekül CD59-Antigen/µm² Plasmamembranoberfläche exprimiert.

16. Zelle nach Anspruch 14. worin die Zelle eine mikrovaskuläre Endothelialzelle ist

17. Zelle nach Anspruch 1, welche eine Mauszelle ist.

18. Zelle nach Anspruch 17, worin die Zelle die Klasse II MHC-Proteine als Ergebnis einer Disruption des invarianten Kettengens der Klasse II MHC-Gene nicht exprimiert.

19. Zelle nach Anspruch 17, worin die Zelle die Klasse 1 MHC-Proteine als Ergebnis einer Disruption des β2-Mikroglobulingens nicht exprimiert.

20. Transgene Maus, enthaltend eine der Zellen nach den Ansprüchen 17 bis 19.

21. Organ einer transgenen Maus, wobei das Organ aus Zellen nach einem der Ansprüche 17 bis 19 gebildet ist.

22. Prothese zur Implantation in ein Tier oder einen Menschen mit Zellen nach einem der Ansprüche 1 bis 16, die daran gebunden sind.

23. Prothese nach Anspruch 22, worin die Prothese ein Gefäßimplantat ist und die Zellen mikrovaskuläre Endothelialzellen sind.

24. Verfahren zur Reendothelialisierung eines freigelegten Blutgefäßes ex vivo, umfassend das Applizieren von Zellen nach Anspruch 16 oder der Prothese nach Anspruch 23 auf das Gefäß.

25. Zelle nach einem der Ansprüche 1 bis 16 oder ein Organ nach Anspruch 21 oder eine Prothese nach den Ansprüchen 22 oder 23 zur Verwendung in der Medizin.

26. Verwendung der Zellen nach Anspruch 16 oder der Prothese nach Anspruch 22 bei der Herstellung eines Mittels zur Reendothelialisierung eines denudierten Blutgefäßes.

## Revendications

1. Cellule de mammifère modifiée génétiquement pour la transplantation dans l'homme ou un animal,
dans laquelle la cellule n'exprime pas sur sa surface des protéines codées par les gènes du complexe majeur d'histocompatibilité de catégorie I ou les gènes du complexe majeur d'histocompatibilité de catégorie II qui engendrent une réponse à médiation par les lymphocytes T contre la cellule, et
la cellule exprime une molécule nucléotidique incorporée de manière stable qui code pour une protéine inhibant l'attaque, à médiation par le complément, de la cellule modifiée génétiquement lors de l'introduction dans un animal d'une autre espèce ou un autre individu.

2. Cellule suivant la revendication 1, dans laquelle la molécule nucléotidique code pour une protéine inhibant l'attaque, à médiation par le complément, dans la même espèce que l'espèce dont la cellule est dérivée, et la molécule nucléotidique est située à un locus distinct de celui du gène naturel codant pour la même molécule dans la cellule.

3. Cellule suivant la revendication 1, dans laquelle le promoteur du gène codant pour un inhibiteur protéique de l'attaque, à médiation par le complément, sur la cellule hôte contenant le gène est modifié de manière à accroître l'expression du gène dans la cellule hôte.

4. Cellule suivant la revendication 1, dans laquelle la protéine inhibant l'attaque, à médiation par le complément, est choisie dans le groupe consistant en CD59, CD46 et CD55.

5. Cellule suivant la revendication 1, choisie dans le groupe consistant en des cellules endothéliales, des fibroblastes, des cellules épithéliales, des cellules des muscles squelettiques, du muscle cardiaque et des muscles lisses, des hépatocytes, des cellules des îlots pancréatiques, des cellules de la moelle osseuse, des astrocytes et des cellules de Schwann.

6. Cellule suivant la revendication 1, dans laquelle la cellule n'est pas d'origine humaine, la protéine inhibant le complément est une protéine humaine et les gènes du CMH de catégorie II sont choisis dans le groupe consistant en l'équivalent de HLA DP, DR et DQ dans l'espèce de cellule.

7. Cellule suivant la revendication 1, dans laquelle la cellule n'est pas d'origine humaine, la protéine inhibant l'attaque, à médiation par le complément, est une protéine humaine et les gènes du CMH de catégorie I sont choisis dans le groupe consistant en l'équivalent de HLA A, B et C dans l'espèce de cellule.

8. Cellule suivant la revendication 1, dans laquelle la cellule n'exprime pas les protéines du CMH de catégorie II en résultat de la rupture du gène de chaîne invariante des gènes du CMH de catégorie II.

9. Cellule suivant la revendication 1, dans laquelle la cellule n'exprime pas les protéines du CMH de catégorie I en résultat de la rupture du gène de β2-microglobuline.

10. Cellule suivant la revendication 1, comprenant en outre une molécule nucléotidique qui est exprimée par la cellule et qui code pour une protéine qui, en présence d'un composé choisi, a pour résultat la mort cellulaire.

11. Cellule suivant la revendication 10, dans laquelle la molécule nucléotidique code pour une cytosine-désaminase bactérienne.

12. Cellule suivant la revendication 1, dans laquelle la cellule est choisie dans le groupe consistant en des cellules d'origine humaine, des cellules d'origine bovine et des cellules d'origine porcine.

13. Cellule suivant l'une quelconque des revendications 1-4 ou 6-12, choisie dans le groupe consistant en des cellules souches hématopoïétiques, la descendance de cellules souches hématopoïétiques et les cellules sanguines matures.

14. Cellule suivant l'une quelconque des revendications 1 à 13, dans laquelle la cellule exprime une molécule nucléotidique qui code pour un agent thérapeutique, qui est sécrété par la cellule et la cellule n'exprime pas normalement la molécule, exprime la molécule à partir du même promoteur ou bien exprime la molécule à partir de locus multiples.

15. Cellule suivant l'une quelconque des revendications 1 à 14, exprimant sur sa surface une quantité supérieure ou égale à 10³ molécules CD59/cellule ou supérieure ou égale à 1 molécule d'antigène CD59/µm² de surface de membrane plasmatique.

16. Cellule suivant la revendication 14, dans laquelle la cellule est une cellule endothéliale microvasculaire.

17. Cellule suivant la revendication 1, qui est une cellule de souris.

18. Cellule suivant la revendication 17, dans laquelle la cellule n'exprime pas les protéines du CMH de catégorie II en résultat d'une rupture du gène de chaîne invariante des gènes du CMH de catégorie II.

19. Cellule suivant la revendication 17, dans laquelle la cellule n'exprime pas les protéines du CMH de catégorie I en résultat d'une rupture du gène de β2-microglobuline.

20. Souris transgénique contenant n'importe laquelle des cellules suivant les revendications 17 à 19.

21. Organe provenant d'une souris transgénique, organe qui est formé de cellules suivant l'une quelconque des revendications 17 à 19.

22. Prothèse pour l'implantation dans un animal ou patient humain, comprenant des cellules suivant l'une quelconque des revendications 1 à 16 fixées à celle-ci.

23. Prothèse suivant la revendication 22, qui est une greffe vasculaire, les cellules étant des cellules endothéliales microvasculaires.

24. Procédé pour la ré-endothélialisation *ex vivo* d'un vaisseau sanguin dénudé, comprenant l'application au vaisseau de cellules suivant la revendication 16 ou de la prothèse suivant la revendication 23.

25. Cellule suivant l'une quelconque des revendications 1 à 16 ou organe suivant la revendication 21 ou bien prothèse suivant la revendication 22 ou 23, destiné à être utilisé en médecine.

26. Utilisation des cellules suivant la revendication 16 ou de la prothèse suivant la revendication 22 dans la production d'un agent destiné à la ré-endothélialisation d'un vaisseau sanguin dénudé.
